# EUROPEAN PATENT APPLICATION

(11) **EP 4 800 094 A1**
(43) Date of publication of application: **02.09.2026**
(21) Application number: 24882444.3
(22) Date of filing: 24.10.2024
(51) Int. Cl.: C12M 3/00, C12M 1/00, C12M 1/36, C12N 5/10, C12N 5/071

(54) **PACKAGE OF CELL PRODUCTION DEVICE, CELL PRODUCTION METHOD, AND MANAGEMENT SYSTEM THEREFOR**

(30) Priority: 26.10.2023 JP 2023184314; 22.07.2024 JP 2024117090
(71) Applicant: CiRA Foundation, Kyoto-shi, Kyoto 606-8397 (JP)
(72) Inventor: HIRANO, Tomoko, Kyoto-shi, Kyoto 606-8397 (JP); SAKAI, Ichiro, Kyoto-shi, Kyoto 606-8397 (JP)
(74) Representative: J A Kemp LLP
(86) International application number: PCT/JP2024/037890
(87) International publication number: WO 2025/089333

(57) **Abstract**

The present invention provides a package that aseptically prepares many types of substances and containers for a cell processing device and reduces the operation for interconnecting the containers, a cell production method using the package, and a management system for producing the package.

## Description

### TECHNICAL FIELD

The present invention relates to a package of a cell production device, a cell production method using the same, a management system for managing the package of the cell production device, and a quality management system for managing a process for producing the package of the cell production device.

### BACKGROUND ART

In recent years, research into regenerative medicine using differentiated cells derived from induced pluripotent stem cells (iPS cells) is currently being actively conducted. In particular, a treatment by which iPS cells are established from a patient's somatic cells (e.g., peripheral blood mononuclear cells or the like) and then various differentiated cells or organoids induced to differentiate from the iPS cells are transplanted into the patient (autotransplantation) has attracted attention as a treatment that can reduce the risk of rejection (Non-Patent Literature 1 and 2). Patent Literature 1 (Japanese Unexamined Patent Publication No. 2017-195905) discloses a system capable of producing stem cells. Also, CAR-T therapy and other treatments using a patient's own somatic cells are beginning to be applied clinically, and Patent Literature 2 (Japanese Unexamined Patent Publication No. 2022-8735) discloses a cell production system and a cell production method, with which costs can be reduced by reducing the number of steps in a treatment method using CAR-T cells.

### Citation List

### Patent Literature

Patent Literature 1: Japanese Unexamined Patent Publication No. 2017-195905
Patent Literature 2: Japanese Unexamined Patent Publication No. 2022-8735
Patent Literature 3: Japanese Unexamined Patent Publication No. 2011-505890

### Non-Patent Literature

Non-Patent Literature 1: Shinsuke Yoshida., et al., CLINICAL AND TRANSLATIONAL RESOURCE AND TECHNOLOGY INSIGHTS VOLUME 4, ISSUE 1, P51-66.E10, JANUARY 13, 2023
Non-Patent Literature 2: Madrid, M., et al., Current Protocols, 1, e88. doi: 10.1002/cpzl.88

### SUMMARY OF INVENTION

### Technical Problem

One of the features of iPS cells is that they can be produced from a patient's own cells, making them an effective means of minimizing the risk of rejection. However, according to the studies of the inventors of the present invention, there are many issues, including cost, that need to be addressed before transplantation therapy using differentiated cells derived from autologous iPS cells can be widely adopted. Specifically, it is essential to develop an automated device that can perform all processes from iPS cell establishment to induction of differentiation in a closed-type device, which has not been possible until now, and to produce high-quality cells while significantly reducing costs by creating kits (packaging) of reagents and materials that can be plugged into the device.

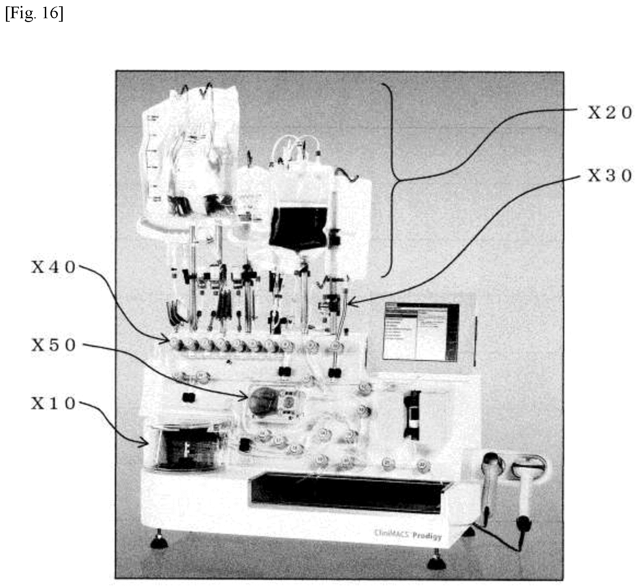

Conventionally, cell processing devices configured to automatically perform sequential processing on cells have been proposed by device manufacturers. Such a cell processing device is configured, as shown in an example in FIG. 16, such that various substances necessary for cell processing are respectively contained in substance supply bags X20, and the substances necessary for processing are supplied sequentially from the substance supply bags X20 into a culture vessel X10. A soft tube X30 extends from each of the numerous substance supply bags X20, and each soft tube X30 passes through a pinch valve X40 that can be controlled to open and close, passes through a peristaltic pump X50, and is connected to the culture vessel X10. The pinch valve and the peristaltic pump are controlled to sequentially feed various substances necessary for the cell processing steps from the respective substance supply bags X20 into the culture vessel X10, and the cells are automatically processed sequentially.

When the conventional cell processing device described above is delivered from the device manufacturer to the user (cell producer), it does not come with supply bags for aseptically storing the various substances necessary for cell production. Therefore, in order to automatically process cells in the intended order using such a cell processing device, the user need to aseptically fill the supply bags X20 with various substances in a safety cabinet prior to processing, aseptically connect them to predetermined positions in the cell processing device, and aseptically attach the soft tube to the pinch valve and the peristaltic pump according to a predetermined piping pattern.

However, it was complicated for users to prepare supply bags in which the many types of reagents, culture media, and the like purchased were aseptically filled into containers, and then place them in the predetermined positions of the cell processing device while ensuring sterility, as this required a large-scale device such as a safety cabinet. The operation of aseptically placing the many types of substance supply bags described above in their predetermined positions is extremely tedious, and it is difficult to eliminate the occurrence of incorrect substance supply bags, incorrect placement positions, and/or incorrect connections of soft tubes, and the like.

In addition to the problem of ensuring sterility as described above, when aiming to achieve both high-quality cell production and significant cost reductions, there is also the problem of the smaller volume of liquid used compared to biopharmaceutical production using a conventional cell processing device. Furthermore, when induced pluripotent stem cells or differentiated cells are produced from patient-derived somatic cells and then autotransplanted into the patient, it is necessary to create a custom-made producing process for each patient and supply the producing materials. Thus, a system of mass production and mass supply using a uniform producing process or materials for all patients is not compatible with cell therapy involving autotransplantation.

It is an object of the present invention to provide a package of a cell production device that can alleviate the above problems. Also, it is another object of the present invention to provide a cell production method using the cell production device of the package. Also, it is another object of the present invention to provide a management system and a quality management system for managing a process for producing the package.

### Solution to Problem

Main configurations of the present invention are as follows.
[1] A package of a cell production device comprising:
   a cell production device; and a packaging material;
   wherein the cell production device has, as a configuration for producing induced pluripotent stem cells,
      a processing container,
      at least one culture medium container for storing a liquid culture medium, and
      a first supply container for storing a fluid containing a reprogramming factor,
   the culture medium container and the first supply container are, such that their contents are fed into the processing container while maintaining a closed system,
      (i) connected to the processing container via a connecting conduit which is switchable between a communication state and a non-communication state, or
      (ii) connectable to the processing container via the connecting conduit and via a unique connector configured to prevent incorrect interconnection,
   the processing container has at least an injection port that is openable and closable for receiving a solution containing somatic cells from an outside, and a discharge port that is openable and closable for discharging the contents,
   the processing container, the culture medium container, and the first supply container are sealed containers, and
   the cell production device is packaged in the packaging material in a sterilized state.
[2] The package of a cell production device according to [1] above,
   wherein the processing container is
      configured to separate somatic cells for use in production of induced pluripotent stem cells from the solution containing somatic cells injected through the injection port, and
      configured to discharge components other than the somatic cells to the outside of the processing container, and leave the somatic cells to be processed inside the processing container, and
   the somatic cells remaining inside the processing container are processed inside the processing container.
[3] The package of a cell production device according to [2] above,
   wherein the processing container is configured to separate somatic cells for use in production of induced pluripotent stem cells through continuous centrifugation.
[4] The package of a cell production device according to any one of [1] to [3] above,
   wherein the cell production device has one or more second supply containers for storing a fluid containing a differentiation-inducing substance for the induced pluripotent stem cells as a configuration for producing differentiated cells from the induced pluripotent stem cells produced in the processing container,
   the second supply containers are sealed containers,
   the second supply containers are, such that their contents are fed into the processing container while maintaining a closed system,
   (iii) connected to the processing container via a connecting conduit which is switchable between a communication state and a non-communication state, or
   (iv) connectable to the processing container via the connecting conduit which is switchable between a communication state and a non-communication state and via a unique connector configured to prevent incorrect interconnection.
[5] The package of a cell production device according to any one of [1] to [4] above,
   wherein one or both of the culture medium container and the first supply container are each separated from the cell production device and packaged in separate packaging materials, and the package is separated into two or more packages that are independent of each other.
[5a] The package of a cell production device according to [5] above,
   wherein one or both of the medium container and the first supply container are packaged in separate packaging materials separate from the cell production device in order to ensure temperature control necessary for the one or both thereof.
[6] The package of a cell production device according to any one of [1] to [5] and [5a] above,
   wherein the cell production device packaged in the packaging material in the package is a closed system processing circuit portion included in the cell processing device for processing the somatic cells,
   the cell processing device is configured to include the closed system processing circuit portion and a device body,
   the device body includes an operating mechanism that operates the closed system processing circuit portion and a control unit that controls the operating mechanism, and
   the closed system processing circuit portion is detachable from the device body while maintaining the closed system, and is assembled as a closed system in a state in which the processing circuit portion is taken out from the device body, and the closed system processing circuit portion attached to the device body is operated by the operating mechanism under control of the control unit, thereby processing the somatic cells inside the closed system processing circuit portion.
[7] The package of a cell production device according to [6] above,
   wherein the cell production device is assembled as a closed system by a device assembler separate from a cell producer who produces cells using the cell processing device, and the cell production device is assembled based on specification data issued by the cell producer, packaged as the package, and provided to the cell producer.
[8] The package of a cell production device according to [7] above,
   wherein the cell producer and the device assembler are different entities.
[9] The package of a cell production device according to [7] or [8] above,
   wherein the specification data includes
      at least information about a donor of the somatic cells,
      information about the device body to which the cell production device is to be attached as a closed system processing circuit portion,
      information about a configuration of the cell production device, and
      the date of use of the cell production device,
   the cell production device displays an identification code that is readable by a reading device, and information about the donor of the somatic cells included in the specification data and information about an actual configuration of the cell production device produced are obtainable from a separately provided management device through the identification code, and
   the information about the actual configuration of the cell production device is stored in a storage device of the management device in association with information about the configuration of the cell production device included in the specification data.
[10] A cell production method using the package of a cell production device according to any one of [1] to [9] above, the cell production method comprising:
   a step of preparing the cell production device taken out from the package; and
   a step of producing induced pluripotent stem cells in the processing container of the cell production device,
   wherein the step of producing the induced pluripotent stem cells includes
   a step (s10) of injecting the solution containing the somatic cells into the processing container through the injection port, feeding the liquid culture medium from the culture medium container into the processing container, and feeding the fluid containing the reprogramming factor from the first supply container into the processing container, thereby bringing the reprogramming factor into contact with the somatic cells in the liquid culture medium in the processing container, and
   a step (s20) of culturing the somatic cells in the liquid culture medium in the processing container to establish induced pluripotent stem cells.
[11] The cell production method according to [10] above,
   wherein the package of a cell production device is the package according to [2] or [3],
   in the step (s10),
      the solution containing the somatic cells is injected into the processing container through the injection port,
      then the somatic cells for use in production of induced pluripotent stem cells are separated in the processing container, and components other than the somatic cells are discharged to an outside of the processing container, the somatic cells to be processed are left inside the processing container, and
      then the liquid culture medium is fed from the culture medium container, and the fluid containing the reprogramming factor is fed from the first supply container, thereby bringing the reprogramming factor into contact with the somatic cells in the liquid culture medium within the processing container.
[12] The cell production method according to [11] above,
   the package of a cell production device being the package of a cell production device according to [4],
   the method further comprising, after the step (s20), a step (s30) of feeding the fluid containing the differentiation-inducing substance for induced pluripotent stem cells from the second supply containers into the processing container, thereby forming differentiated cells in the processing container.
[13] A management system for the package of a cell production device according to any one of [1] to [9] above,
   the management system comprising a management device; and a reading device,
   wherein one or both of the cell production device and the packaging materials surrounding the cell production device in all the packages produced displays an identification code (D1) of each package so as to be readable by the reading device,
   the reading device is for reading the identification code,
   the management device has a data receiving unit and an attribute information storage unit,
   the data receiving unit receives the identification code read by the reading device as input data,
   the attribute information storage unit stores the respective identification codes (D1) of all the packages to be produced and respective pieces of product attribute information (P1) in a mutually associated state, and
   the management device is configured to refer to the identification code (D1) received by the data receiving unit, and the identification codes (D1) and the pieces of product attribute information (P1) stored in the attribute information storage unit, and output the product attribute information (P1) associated with the read identification code (D1).
[14] The management system according to [13] above,
   wherein an identification code (D10) of the cell production device is displayed on the cell production device to be readable by the reading device,
   the product attribute information (P1) of each of all the packages stored in the attribute information storage unit includes the identification code (D10) of the cell production device of the package and product attribute information (P10) thereof in a mutually associated state, and
   the management device is configured to refer to the identification code (D10) received by the data receiving unit, and the identification codes (D10) and the pieces of product attribute information (P10) stored in the attribute information storage unit, and output the product attribute information (P10) associated with the received identification code (D10).
[15] The management system according to [14] above,
   wherein each sealed container for storing a substance in the cell production device displays an identification code (D20) of the substance stored in the sealed container such that the identification code (D20) is readable by the reading device,
   the product attribute information (P10) of each cell production device in the packages includes an identification code (D20) of each substance stored in each sealed container and product attribute information (P20) thereof in a mutually associated state, and
   the management device is configured to refer to the identification code (D20) received by the data receiving unit, and the identification codes (D20) and the pieces of product attribute information (P20) stored in the attribute information storage unit, and output the product attribute information (P20) associated with the received identification code (D20).
[16] The management system according to [14] or [15] above,
   wherein components constituting the cell production device display identification codes (D30) of the corresponding components such that the identification codes are readable by the reading device,
   the product attribute information (P10) of cell production devices in the packages includes identification codes (D30) of the components constituting the cell production device and pieces of product attribute information (P30) thereof in a mutually associated state, and
   the management device is configured to refer to the identification code (D30) received by the data receiving unit, and the identification codes (D30) and the pieces of product attribute information (P30) stored in the attribute information storage unit, and output the product attribute information (P30) associated with the received identification code (D30).
[17] The management system according to any one of [14] to [16] above,
   wherein each container storing the solution containing somatic cells to be processed in the cell production device displays a code (D2) of the somatic cells in the container or a donor of the somatic cells such that the code is readable by the reading device,
   the attribute information storage unit stores the code (D2) and the corresponding attribute information (P2), and the code (D2) and the corresponding attribute information (P2) are associated with each other and with an identification code (D1) of a package for use in processing or an identification code (D10) of the cell production device in the package, and
   the management device is configured to refer to the code (D2) received by the data receiving unit, and the codes and the attribute information stored in the attribute information storage unit, output the attribute information (P2) associated with the received code (D2), and output the identification code (D1) of the package for use in processing or the identification code (D10) of the cell production device in the package.
[18] The management system according to any one of [14] to [17] above,
   wherein the attribute information storage unit stores a code (D3) of a patient to whom cells produced by the cell production device need to be applied and attribute information (P3) thereof, and the code (D3) of the patient and the attribute information (P3) thereof are associated with each other and with an identification code (D1) of a package for use in processing or an identification code (D10) of the cell production device in the package, and
   the management device is configured to refer to the identification code (D1) or the identification code (D10) received by the data receiving unit, and the identification code and the attribute information stored in the attribute information storage unit, and output the code (D3) of the patient to whom the produced cells are to be applied and the attribute information (P3).
[19] The management system according to any one of [14] to [18] above,
   wherein the cell production device in the package is a detachable closed system processing circuit portion in a cell processing device including the detachable closed system processing circuit portion, a mechanism that operates the processing circuit portion, and a control unit,
   an identification code (D11) of the cell processing device is displayed on a portion of the cell processing device excluding the closed system processing circuit portion to be readable by a reading device,
   the attribute information storage unit stores the identification code (D11) of the cell processing device and product attribute information (P11) thereof,
   the identification code (D11) of the cell processing device and the product attribute information (P11) thereof are stored in the attribute information storage unit in association with each other and in association with the identification code (D1) of the package for use in processing or the identification code (D10) of the cell production device in the package, and
   the management device is configured to refer to the identification code (D1) or the identification code (D10) received by the data receiving unit, and identification codes and product attribute information stored in the attribute information storage unit, and to output an identification code (D11) and product attribute information (P11) thereof.
[20] The management system according to any one of [13] to [19] above,
   wherein the cell production device in the package is a detachable closed system processing circuit portion in a cell processing device including the detachable closed system processing circuit portion, a mechanism that operates the processing circuit portion, and a control unit,
   the management device further includes a specification data storage unit, the specification data storage unit stores specification data (S1a) including at least
      attribute information of the solution containing somatic cells for use or the donor,
      product attribute information of the package for use or the cell production device thereof,
      the date of use of the cell production device, and
      identification information of the cell processing device in which the cell production device is installed as the processing circuit portion, and
   the management system includes a data input device for inputting the specification data, and
   the package is assembled based on the specification data (S1a),
   the management device is configured to receive, via the data receiving unit,
      specification data (S1b) including at least
      attribute information of the solution containing somatic cells for use or the donor,
      product attribute information of the package for use or the cell production device thereof,
      information about the date when the cells are produced, and
      identification information of the cell processing device for use, the attribute information, the product attribute information, the information about the date, and the identification information being entered through the reading device by the cell producer on the date when the cells are produced,
         and,
      to output a signal indicating a match when the specification data (S1b) received by the data receiving unit matches the specification data (S1a) stored in the specification data storage unit.
[21] The management system according to [20] above,
   wherein the control unit of the cell processing device is configured to receive a signal indicating the match output from the management device, and is configured to enable start of the cell processing device only when the signal is received.
[22] A quality management system for the cell production device in the package according to any one of [7] to [9] above,
   wherein a process for producing the package includes
      an element producing process for each of a plurality of elements constituting the cell production device in the package, in which each element is produced by an element manufacturer in the respective technical field of the element, and
      a device assembly process in which the elements produced in the element producing process are collected and the device assembler assembles the elements into the cell production device,
   the quality management system includes
      a process management device and
      a plurality of terminal devices connected to the process management device to enable data communication,
   the terminal devices are arranged to be used by the cell producer who is a user of the cell production device, the device assembler, and the element manufacturer,
   the cell production device is assembled based on specification data indicating a configuration of the cell production device,
   the specification data is designed by the cell producer, who has received a production order from a medical institution to produce target cells for patient treatment, such that the cell production device has a configuration for producing the target cells and is adapted to the cell processing device selected to produce the target cells, and
   the specification data is associated with an identification code of the patient,
   the process management device
      receives the specification data and the identification code of the patient associated therewith from the cell producer or the device assembler via the terminal device and stores them in a storage area,
      allocates a storage area for storing actual specification data corresponding to the specification data in the cell production device that is actually produced,
      receives, from the element manufacturer, actual data for each element produced by the element manufacturer who has received the production order from the device assembler based on the specification data, and stores the actual data in the storage area for storing actual specification data,
      receives data regarding an overall arrangement of the cell production device from the device assembler who has assembled the cell production device using each element delivered from the element manufacturer and stores the data in the storage area for storing the data, and
      determines whether the original specification data and the actual specification data match, and outputs the determination result.
[23] The quality management system according to [22] above,
   wherein the terminal devices are each equipped with a function of reading an identification code, and
   identification codes indicating respective pieces of identification information are respectively displayed on surfaces of the packaging materials of the packages and on the elements of the cell production device.
[24] The quality management system according to [23] above,
   wherein the identification codes are each displayed in the form of a two-dimensional symbol.
[25] The quality management system according to any one of [22] to [24] above,
   wherein the cell producer, the device assembler, and the element manufacturer are different entities.

### Advantageous Effects of Invention

In the package of the cell production method according to the present invention (hereinafter also referred to as the package), and the cell production method using the same, each sealed container for storing a predetermined substance is provided to the user (cell producer) in a state in which the sealed containers are pre-connected to the cell processing container, or in a state in which they are not connected but can be connected via a unique connector configured to prevent incorrect interconnection. This configuration prevents a sealed container for storing an incorrect substance from being connected to the cell processing container when a user produces cells.

In addition, in a preferred embodiment of the present invention, when a cell producer performs cell processing using a predetermined cell processing device, the device assembler is assigned to assemble the closed system processing circuit portion (the cell production device according to the present invention) having components necessary for the cell processing and provide the processing circuit portion to the cell producer. In response to a request from the cell producer, the device assembler collects the parts and elements necessary for the closed system circuit portion from various element manufacturers, assembles the closed system processing circuit portion (hereinafter also referred to as the "closed system portion") to form a package, and sends the package to the cell producer.

Conventionally, cell producers have directly ordered the various elements that constitute the closed system portion from various element manufacturers that specialize in the manufacture of each element, and then collect and assembled them themselves. In contrast, in the present invention, a device assembler is appointed between the cell producer and the element manufacturer, and thus the cell producer can obtain the closed system portion on the day of cell production or an appropriate date before that by simply informing the device assembler of the configuration of the closed system portion, and is free from the hassle and errors involved in assembling the closed system portion.

Furthermore, by appointing the device assembler, it is possible to achieve both high-quality cell production and significant cost reductions from the viewpoint of ensuring the volume of reagents and sterility. By having a dedicated device assembler design the flow path system, select bags, and design the amount of reagents, it is possible to reduce the volume of liquid used compared to the volume of liquid used in cell production with conventional biopharmaceutical production devices. In particular, in the production of patient-derived autologous iPS cells, rather than mass-producing the package of the present invention uniformly for all patients, the package of the present invention can be optimally designed and produced individually for each cell producing process (custom-made) depending on the differentiated cells required by the patient, which are the final production result, and the composition of the patient's blood, which serves as the raw material. For example, depending on the differentiated cells required by the patient, which are the final production result, additional induction factors may be added to the bag, or the amount of culture medium may be increased or reduced to increase or reduce the number of days of culture. In addition, when it is expected that the composition of the patient's blood, which serves as the raw material, makes it difficult to establish iPS cells using standard protocols, it may be possible to optimize the package, for example, by increasing the amount of reprogramming factors and the like. Furthermore, the package according to the present invention is single-use and disposable, but eliminating package waste by the device assembler can reduce the environmental impact.

The management system according to the present invention enables tracking (traceability) of the processes for producing materials and mechanical components that constitute the package and the raw materials, and also enables management of the relationship between the package and the somatic cells (and donors thereof).

The quality management system according to the present invention manages the elements and components that are delivered to the device assembler in accordance with the specification data issued by the cell producer. This allows cell producers to produce cells without errors. In addition, medical institutions can obtain target cells obtained from the patient's blood provided to the cell producer.

### Brief Description of Drawings

[FIG. 1] FIG. 1 shows a block diagram showing a configuration example of a package of a cell production device according to the present invention. Each thick black line, typically indicated by reference symbol A10, indicates a connecting conduit such as a soft tube.
[FIG. 2] FIG. 2 shows a block diagram showing another configuration example of the package of a cell production device according to the present invention.
[FIG. 3] FIG. 3 shows a block diagram showing an example of a feeding device in the package of a cell production device according to the present invention.
[FIG. 4] FIG. 4 is a photograph showing a device body of a commercially available automatic cell processing device, CliniMACS Prodigy available from Miltenyi Biotec, from which a closed system portion (the cell production device in the present invention) has been removed.
[FIG. 5] FIG. 5 is a photograph showing an example of a package accommodating a closed system portion (the cell production device in the present invention) to be attached to the device body of CliniMACS Prodigy shown in FIG. 4. In the example shown in FIG. 5, the lid of the packaging material that constitutes the package has been removed to show the inside of the package.
[FIG. 6] FIG. 6 is a photograph showing an example of a blood collection device to be connected to the cell production device in the present invention.
[FIG. 7] FIG. 7 is a photograph illustrating a state in which the closed system portion (the cell production device in the present invention) is attached to the device body of the CliniMACS Prodigy shown in FIG. 5. The closed system portion in FIG. 7 is shown to illustrate the attached state, and is not the same as the closed system portion shown in FIG. 6.
[FIG. 8] FIG. 8 is a photograph showing a device body of a commercially available automatic cell processing device, the Gibco CTS Rotea Counterflow Centrifugation System from Thermo Fisher Scientific, from which the closed system portion (the cell production device in the present invention) has been removed.
[FIG. 9] FIG. 9 is a photograph showing an example of a package accommodating a closed system portion (the cell production device in the present invention) to be attached to the device body of the Gibco CTS Rotea Counterflow Centrifugation System shown in FIG. 8. In the example shown in FIG. 9, the lid of the packaging material that constitutes the package has been removed to show the inside of the package.
[FIG. 10] FIG. 10 is a photograph illustrating a disposable unit called a single-use kit, which engages with and operates on the device body, of the closed system portion (the cell production device in the present invention) shown in FIG. 9. The photograph of the single-use kit is an advertisement photograph issued by the manufacturer of the cell processing device.
[FIG. 11] FIG. 11 is a photograph illustrating a state in which a closed system portion (the cell production device in the present invention) is attached to the device body of the Gibco CTS Rotea Counterflow Centrifugation System shown in FIG. 8. The circuit portion of the closed system in FIG. 11 is shown to illustrate the attached state, and is not the same as the closed system portion shown in FIG. 9.
[FIG. 12] FIG. 12 is a photograph showing an example of a configuration of the packaging material of the package.
[FIG. 13] FIG. 13 shows a block diagram showing a configuration example of the management system according to the present invention. In FIG. 13, the dashed lines indicate wired or wireless communication paths.
[FIG. 14] FIG. 14 is a block diagram illustrating the flow of orders and items in the process for producing a package in the present invention. In FIG. 14, the flow of orders and deliveries is indicated by arrows.
[FIG. 15] FIG. 15 shows a block diagram showing a configuration example of a quality management system according to the present invention. In FIG. 15, the dashed lines indicate wired or wireless communication paths.
[FIG. 16] FIG. 16 shows a photograph showing a configuration example of a conventional cell production device.

### DESCRIPTION OF EMBODIMENTS

### 1. Package of Cell Production Device

First, a package of a cell production device according to the present invention will be described.

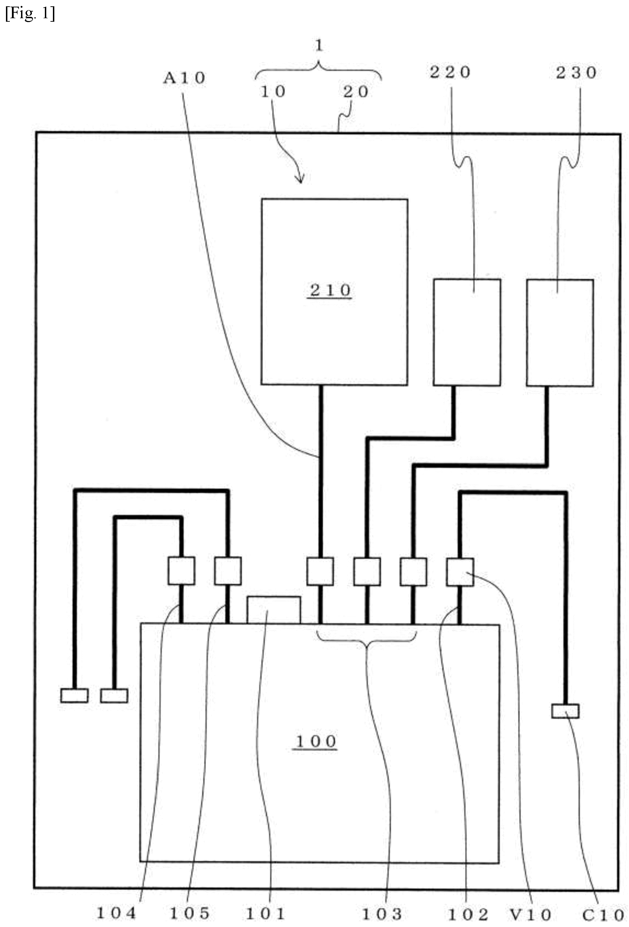

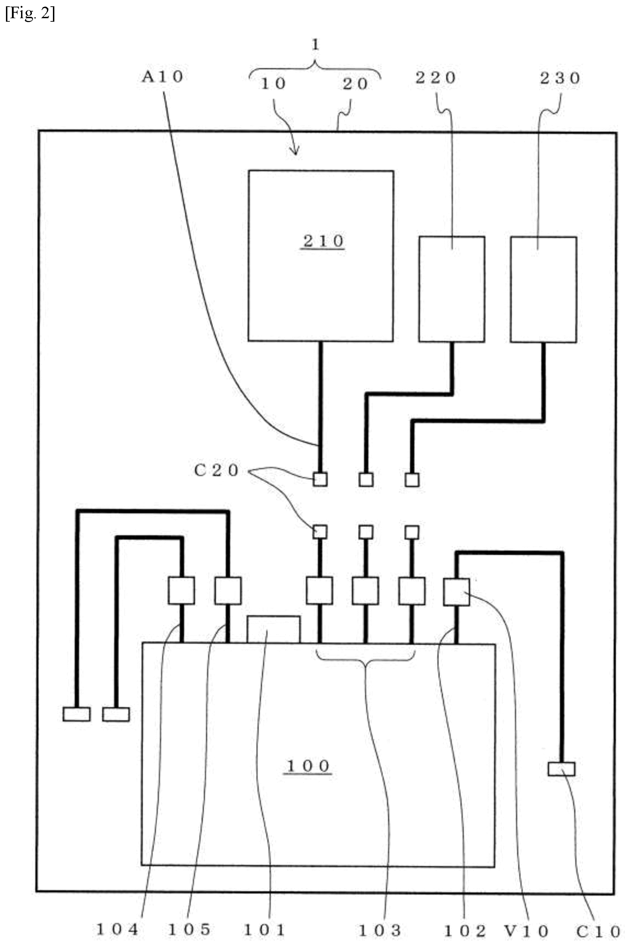

Hereinafter, the cell production device that constitutes the package will be also referred to as the "production device" or "production device of the package". As illustrated in FIG. 1, a package 1 includes a production device 10 and a packaging material 20. The production device 10 is either in a state in which sealed containers (210 to 230) described below have been connected to a processing container 100, or in a state in which the sealed containers are not connected but can be unambiguously connected using connectors that prevent incorrect interconnection, as illustrated in FIG. 2. Furthermore, the production device 10 is packaged in the packaging material 20 to form the package 1. In the production device 10, the inside of the production device (the inside of the flow path, the inside of each container, contents, and the like) that affects cell processing needs to be in a sterile state to an extent that it is suitable for cell processing. However, in a preferred embodiment, not only inside but also on the outer surface of the production device 10 is sterilized, and the production device 10 is packaged in the packaging material 20 such that the sterilized state of the outer surface is maintained, forming the package 1. In a preferred embodiment, the production device of the package does not have a feeding device, electromagnetic opening and closing valve, control device, power supply device, or the like (it may include a pinch cock that is openable and closable manually or the like), and is configured as a closed system portion (closed system processing circuit portion) that can be attached to and detached from the cell processing device (apparatus)such as that shown in FIG. 16. The "circuit" in the "processing circuit portion" here does not necessarily have to have a circulation flow path, but also includes a flow path configuration in which the processing container and a predetermined container are simply connected via a connecting conduit (or a flow path configuration that can be connected to the processing container via a unique connector). Examples of such cell processing devices include CliniMACS Prodigy available from MiltenyiBiotec, Gibco CTS Rotea Counterflow Centrifugation System available from Thermo Fisher Scientific, Cocoon system available from Lonza, and Xuri, which is a Cell Expansion System available from Cytiva. Therefore, the production device 10 is ready to be provided to the user of the cell processing device, who is the cell producer, and the user can simply remove the production device from the package and attach it to the cell processing device as a closed system portion, thereby starting cell production without having to work in a sterile environment in a safety cabinet and without a conventional complicated connection operation, or the connection operation performed by the user can be reduced. As will be described in detail below, in a preferred embodiment of the present invention, a device assembler is newly appointed to assemble the package, and the package assembled by the device assembler is provided to the user.

### (Configuration for Producing iPS cells)

The production device 10 has at least a processing container 100, a culture medium container 210, and a first supply container 220 as a configuration for producing iPS cells from somatic cells. The processing container 100 is a sealed container for processing somatic cells injected from the outside. The culture medium container 210 is a sealed container for storing a liquid culture medium. The first supply container 220 is a sealed container for storing a fluid containing a reprogramming factor for establishing iPS cells. The "sealed containers" will be described later in detail.

In the present invention, a "sealed container" refers not only to a container that is closed airtight or liquidtight, but also to a container whose interior is closed to an extent that, when each port or pipe is closed, microorganisms or viruses cannot enter from the outside, i.e., to an extent that sterility within the container is maintained. For example, a closed container, in which porous filters (e.g., a pore size of about 0.2 µm or less, particularly about 0.1 to 0.2 µm) are provided at inlet and outlet ports that allow permeation of a fluid (particularly gases) but do not allow permeation of bacteria or viruses, allows permeation of outside air through the porous filter and the entry of the outside air into the container, but bacteria and viruses cannot enter the container, and the sterility of the closed container is thus maintained, making it a sealed container. A closed container whose wall is a gas-permeable membrane that prevents permeation of bacteria, viruses, and the like but allows permeation of O₂ gas molecules and CO₂ gas molecules is also a sealed container. Similarly, a "closed system" does not only refer to a system that is airtight or liquidtight, but also to a system that is closed to an extent that microorganisms or viruses cannot enter from the outside, i.e., to an extent that sterility within the system is maintained.

The culture medium container 210 and the first supply container 220 are connected or connectable to the processing container such that their contents are fed to the processing container while maintaining the closed system. As illustrated in FIG. 1, the culture medium container 210 and the first supply container 220 are connected to the processing container 100 via a connecting conduit A10 that can be switched between a communication state and a non-communication state, eliminating the opportunity for the user to make the connection. Alternatively, as illustrated in FIG. 2, the culture medium container 210 and the first supply container can be connected to the processing container 100 via a unique connector C20 configured to prevent incorrect interconnection. In the example shown in FIG. 2, for a brief description, three pairs of connectors C20 are represented by the same square symbol, but pairs of connectors have different specifications from each other and cannot be connected to each other. In the example shown in FIG. 2, the culture medium container 210 and the first supply container 220 are not connected to the processing container 100, but are always pre-connected via a unique connector C20. Note that in the example shown in FIG. 2, each connecting conduit A10 can be switched between a communication state and a non-communication state.

### (Processing Container)

The processing container 100 has at least an injection port 101 that is openable and closable for receiving a solution containing somatic cells from the outside, and a discharge port 102 that is openable and closable for discharging the contents. A variety of sealed containers may be connected to the injection port 103. As shown in the example of FIG. 1, in a preferred embodiment, the processing container 100 may further have a gas injection port 104 for injecting gases used for cell culture, such as CO₂, nitrogen, oxygen, and air for discharging the contents, and a gas discharge port 105 for releasing gas to prevent pressure increase inside the container. A sterile filter may be provided on the gas cylinder side and a sterile filter may be provided on the gas injection port 104. The gas discharge port 105 may be provided with a sterile filter. A sensing probe may be provided at the processing container or at a leading end on the discharge side of the discharge port 102, and the oxygen concentration and pH of the contents are measured by the sensing probe.

In this specification, "induced pluripotent stem cells (iPS cells)" are cells obtained by reprogramming mammalian somatic cells or undifferentiated stem cells by introducing reprogramming factors. Induced pluripotent stem cells can differentiate into tissues and cells with a variety of different morphologies and functions in the body, and have the ability to differentiate into cells of any of the three germ layers (endoderm, mesoderm, and ectoderm).

In this specification, the induced pluripotent stem cells may be cells derived from patients. Generating iPS cells from patient-derived somatic cells for clinical use could be an effective way to minimize the risk of rejection.

The package of the cell production device according to the present invention can be used with any currently available induced pluripotent stem cells. The concept of the package of the cell production device according to the present invention can also be used to CAR-T cells.

In this specification, the induced pluripotent stem cells may be cells derived from patients with hereditary diseases. Since cells obtained through induction of differentiation of pluripotent stem cells derived from patients with hereditary diseases can serve as disease models that reflect the pathology of such diseases, the cells are suitable for applications such as screening of therapeutic or preventive agents for the diseases. Alternatively, by repairing the gene through genome editing, such as the CRISPR-Cas system, in pluripotent stem cells derived from patients with hereditary diseases, and then differentiating them into target cells, it is also possible to use the cells as therapeutic medicament for the diseases.

The "somatic cells" in the present invention refer to the original cells to be processed inside the processing container 100. In this specification, the "somatic cells" refers to cells that constitute an animal, other than germ cells. Somatic cells are not particularly limited and include both mature healthy and diseased somatic cells, as well as primary culture cells, passaged cells, and established cell lines. Specifically, somatic cells may be, for example, suspension cells (e.g., blood cells or the like) or adherent cells, but are preferably suspension cells. Examples of somatic cells used in the production method according to the present invention include, but are not limited to, skin fibroblasts, mesenchymal stem cells derived from skin cells, visual cells, brain cells, hair cells, oral mucosa, dental pulp cells, lung cells, liver cells, gastric mucosa cells, intestinal cells, spleen cells, pancreatic cells, kidney cells, neural stem cells, wisdom teeth, and the like, tissue stem cells, tissue progenitor cells, blood cells (e.g., hematopoietic stem cells, peripheral blood mononuclear cells (PBMCs) (including T cells and non-T cells), umbilical cord blood cells, and the like), epithelial cells, endothelial cells (e.g., vascular endothelial cells), and muscle cells.

In one embodiment, when blood cells (e.g., peripheral blood mononuclear cells) are used as somatic cells, the cells can be obtained from whole blood through centrifugation (such as density gradient centrifugation or specific gravity centrifugation), separation using a filter (such as a leukocyte removal filter), or separation using an antibody, a magnetic body (such as magnetic beads), or separation using a hydrophilic polysaccharide (such as Ficoll^{™}).

In this specification, the species from which the somatic cells are derived is not particularly limited, and the preferred species from which the somatic cells are derived is human.

In this specification, unless otherwise specified, the term "cell" includes a "cell population". The cell population may be constituted by one type of cell, or two or more types of cells.

In this specification, "a solution containing somatic cells" refers to raw materials such as whole blood, urine (which contains cells), or tears, dilutions or concentrates of such raw materials, liquids containing cells that are still being processed, or liquids containing somatic cells or processed cells that have undergone cell separation processing.

In this specification, "processing" cells means subjecting cells to treatments such as culturing of the cells, dilution of a solution containing the cells, washing of the cells, and separation of the target cells from a solution containing the cells. Also, it also means subjecting cells to chemical treatment, modification of biological properties, combination with non-cellular components, or genetic engineering modification for the purpose of artificial proliferation and differentiation of cells, establishment of cell lines, activation of cells, or the like.

A configuration of the processing container 100 is not particularly limited, but examples thereof include:
(i) a sealed container made of a hard material,
(ii) a sealed container made of a flexible material,
(iii) a sealed container made of a composite material of a flexible material and a hard material, and the like.

A connection structure and material of the port portion provided in the processing container 100 may be appropriately selected with reference to conventionally known sealed containers for cell culture.

### (Sealed Container Made of Hard Material)

The sealed container (i) made of the hard material is preferably strong enough to be unlikely to deform under the weight of the container storing the solution containing somatic cells, and may be one designed and manufactured specifically for the present invention. When the production device 10 is used as a closed system processing circuit portion that can be attached to and detached from a commercially available cell processing device (such as CliniMACS Prodigy (registered trademark)), it is preferable that the processing container 100 is a direct conversion of the processing container (removable) originally provided in such a commercially available cell processing device, or a compatible product that has been separately produced.

As the hard material, it is possible to use known materials that have traditionally been used for hard containers for cell culture, such as glass and plastic materials (e.g., polycarbonate, polyester, polyamide, polystyrene, acrylonitrile-butadiene-styrene copolymer (ABS resin), polyethylene, polypropylene, polyvinyl chloride, biodegradable resin).

### (Sealed Container Made of Flexible Material)

The structure of the sealed container (ii) made of the flexible material is not particularly limited. Typical examples include soft bags and bottles made of flexible films or flexible sheets, which are used for conventionally known cell culture bags, transfusion bags, and the like. The flexible film is a film that has flexibility to an extent that it can deform according to the amount of contents in the bag. Although a configuration of the bag is not limited, an example thereof is a bag structure in which two rectangular or square flexible films are stacked together and the outer peripheral edges, excluding the opening and the inlet and outlet ports, are fused (by heat fusion, high-frequency fusion, or the like) or attached together. The flexible film is preferably gas permeable, allowing the permeation of O₂ and CO₂ necessary for cell culture. If the flexible film is gas impermeable, O₂ and CO₂ necessary for cell culture can be supplied as appropriate through a gas injection port or the like. Furthermore, it is preferable that the flexible film is made of a material that has excellent industrial moldability, can withstand gamma ray sterilization, and is transparent such that the state of the culture medium inside can be observed. An additional structure such as a hole for suspending the bag can be provided as appropriate by referring to conventionally known cell culture bags. A sterile filter can be connected to the bottle. The sterile filter used may be a porous filter (e.g., one with a pore size of about 0.2 µm or less, particularly about 0.1 to 0.2 µm). Here, the sealed container may have an air hole. The sealed container may have a dispensing hole.

The flexible film may be made of known materials used for cell culture bags or the like, such as polyethylene, polyethylene terephthalate, polypropylene, ethylene vinyl acetate copolymer (EVA), ultra-low density polyethylene (ULDPE)/ethyl vinyl alcohol (EVOH), ultra-low density polyethylene (ULDPE), polyolefin (PO), tank liners, polyvinylidene fluoride, and polyethersulfone. The flexible film may be a single layer film or a multi-layer film made of these materials.

### (Processing Container Made of Composite Material)

An example of the sealed container (iii) made of a composite material of the flexible material and the hard material is a container whose framework is made of a hard material and whose wall is made of a flexible film. Such containers have the three-dimensional characteristics of hard containers, but are inexpensive and disposable.

### (Volume of Processing Container)

Although the volume of the processing container is not particularly limited, for applications such as transplantation therapy using differentiated cells derived from autologous iPS cells, it is preferably about 10 to 2000 ml, and more preferably about 200 to 250 ml.

The processing container does not necessarily need to be supplied to the user in an empty state, and a liquid culture medium or the like may be stored in the processing container from the beginning, in addition to the contents of each sealed container.

### (Function to Separate Somatic Cells)

In a preferred embodiment of the present invention, the processing container 100 is configured to separate somatic cells for use in the production of iPS cells from a solution containing somatic cells injected through an injection port, and is configured to discharge components other than the separated somatic cells to the outside of the processing container 100 through the discharge port 102 or the like, leaving the somatic cells to be processed inside the processing container. In this case, the solution containing somatic cells to be injected into the processing container 100 may be the raw material such as whole blood or urine, or a dilution or concentrate of the raw material.

### (Supply of Solution Containing Somatic Cells)

The solution containing somatic cells may be injected from a syringe, an infusion bag, or the like connected to the openable and closable injection port 101 shown in FIG. 1. When producing induced pluripotent stem cells for autotransplantation using whole blood from a patient as a solution containing somatic cells, the amount of whole blood supplied to the device is not particularly limited, but may be about 1 to 200 ml.

Although the method and configuration for separating the target somatic cells from a solution containing somatic cells inside the processing container 100, discharging unnecessary materials, and leaving the somatic cells inside the processing container 100 are not particularly limited, examples thereof include the following:
A separation method in which the target somatic cells are labeled with magnetic beads and then magnet-attracted, leaving the somatic cells in the processing container; and a separation method in which the cells are passed through a microchannel for cell separation; and
A configuration in which continuous centrifugation is carried out as described below.

### (Configuration of Processing Container for Performing Continuous Centrifugation)

In a preferred embodiment, the processing container 100 is configured to perform continuous centrifugation. Configurations for performing continuous centrifugation include configurations for performing various types of centrifugation (so-called counterflow centrifugation, continuous flow centrifugation, zonal centrifugation, and the like) with different detailed configurations, but all of them are centrifuges configured to continuously (or while controlling the flow and blocking with a valve) feed a solution containing somatic cells into a processing container (centrifuge chamber), centrifuge the solution inside the processing container, and continuously (or while controlling the flow and blocking with a valve) discharge the separated unnecessary liquid, leaving the target somatic cells inside the processing container (or discharging the target somatic cells outside the processing container).

A configuration of the processing container for performing such continuous centrifugation can refer to the configuration of conventionally known continuous centrifuges, such as the centrifuges incorporated as part of the closed system in the CliniMACS (registered trademark) Prodigy, which is a cell processing device available from MiltenyiBiotec, Gibco CTS Rotea Counterflow Centrifugation System, which is a cell processing device available from Thermo Fisher Scientific, and the like. The centrifuge mounted on the CliniMACS Prodigy is described in detail in, for example, Patent Literature 3. CliniMACS Prodigy and Gibco CTS Rotea differ in the structure of the rotating portion used for centrifugation, but both have intake and discharge flow paths connected to the processing container, and perform continuous centrifugation (or while controlling conduction and interruption) while the piping remains connected through the action of a rotary joint.

The materials (including the centrifugation medium) necessary for the separation of somatic cells inside the processing container 100 may be injected from the outside as appropriate, and the ports necessary for the injection may be provided as appropriate. It is preferable to adopt an embodiment in which, similar to the culture medium container 210 and the first supply container 220, the container for storing the substance is connected to the processing container 100 in advance via a connecting conduit A10 that can be switched between a communication state and a non-communication state, when injecting the above-mentioned substance into the processing container 100. It is also preferable to adopt an embodiment in which the container for storing the substance is connectable to the processing container via the connecting conduit A10 that can be switched between a communication state and a non-communication state and via a unique connector configured to prevent incorrect interconnection.

The solution containing somatic cells may be one that has been previously processed by an external cell separation device (i.e., a suspension that selectively contains only somatic cells), or, for example, a raw material (such as whole blood or urine) may be processed into a liquid culture medium containing somatic cells. In this case, any conventionally known cell separation device can be used. Also, in this case, the solution containing somatic cells may be injected from a syringe, an infusion bag, or the like connected to the openable and closable injection port 101 shown in FIG. 1. In the processing container, the process can begin with the step of supplying reprogramming factors without the step of cell separation.

### (Culture Medium Container)

The culture medium container 210 as illustrated in FIGS. 1 to 3 is a sealed container for storing a liquid culture medium. Although the form of the sealed container may be any of the forms described in the description of the processing container, when the production device 10 is used as a closed system processing circuit portion that is attached to and detached from a commercially available cell processing device (such as CliniMACS Prodigy (registered trademark)), the sealed container (ii) made of the flexible material (a flexible bag used for cell culture bags, infusion bags, and the like) is preferable as the culture medium container 210.

The liquid culture medium contained in the culture medium container 210 can be used not only for culturing cells, but also for various purposes in the producing process, such as washing of cells and dilution of chemical solutions. Examples of the liquid culture medium that can be used in the present invention include the followings.

A liquid culture medium is not particularly limited, but examples thereof include Essential8 culture medium (CTS^{™} Essential 8^{™} Medium, Essential 8^{™} Medium, Essential 8^{™} Flex Medium, Essential 6^{™} Medium) (Thermo Fisher Scientific Inc.), StemFit (registered trademark) AK02 culture medium (Ajinomoto Co., Inc.), StemFit (registered trademark) AK03 culture medium (Ajinomoto Co., Inc.), StemFit (registered trademark) Basic03 culture medium, CTS (registered trademark) KnockOut SR XenoFree Medium (Gibco), mTeSR1 culture medium, TeSR1 culture medium (Stem Cell Technologies), Iscove's modified Dulbecco's medium (GE HealthCare Technologies Inc.), Improved MEM (Thermo Fisher Scientific Inc.), and the like. These culture media can also be used for culture under feeder-free and xeno-free conditions. Also, examples thereof include, but are not limited to, MSCBM-CD, MSCGM-CD (both are manufactured by Lonza), and mixtures thereof.

Physiologically active substances, nutritional factors, and the like necessary for cell survival or proliferation can be added as needed to the culture medium.

Furthermore, antibiotics such as kanamycin, streptomycin, penicillin, or hygromycin may be added to the culture medium, if necessary.

In this specification, known serum can be used in the culture medium.

In this specification, the culture medium may or may not contain serum substitutes as well as serum.

In this specification, the culture medium may contain a scaffold material (hereinafter also referred to as "scaffold") used for floating culture of cells, and the scaffold material refers to a material or substrate that functions as a scaffold for cells in cell culture. The scaffold material is not particularly limited as long as it can be used for cell floating culture as described above (in other words, it may be free in the culture medium), but examples thereof include those containing or made of synthetic resin, and those made of flexible materials such as collagen. Microcarriers may also be used as scaffold materials. As an example, the scaffold material may include atelocollagen. Typically, the scaffold material is a material other than nanofibers. Examples of polymers used for scaffold materials include polystyrene, polyolefin, polyethylene terephthalate, polyether, polyvinyl alcohol, polyvinyl acetal, polyester, poly(meth)acrylic acid ester, epoxy resin, polyamide, polyimide, polyurethane, polycarbonate, cellulose, dextran, and polypeptide (such as gelatin). The scaffold material may be produced using a known method, or a commercially available product may be used. Examples of commercially available products include Cytodex-1 (GE Healthcare) and Corning (registered trademark) Low Concentration Synthemax (registered trademark) II Microcarriers (Corning).

### (First Supply Container)

The first supply container 220 as illustrated in FIGS. 1 to 3 is a sealed container for storing a fluid containing a reprogramming factor necessary for establishing iPS cells. Although the form of the sealed container may be any of the forms described in the description of the processing container, when the production device 10 is used as a closed system processing circuit portion that is attached to and detached from a commercially available cell processing device (such as CliniMACS Prodigy (registered trademark)), the sealed container (ii) made of the flexible material (a flexible bag used for cell culture bags, infusion bags, and the like) is preferable as the first supply container 220.

In this specification, examples of "reprogramming factors" include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, ESrrb, Nr5a2, Tbx3, and Glis1, and these reprogramming factors may be used alone or in combination. Any known combination of reprogramming factors can be used.

The reprogramming factor introduced into the somatic cells may be in the form of a protein, a nucleic acid (RNA or DNA) encoding the protein, or an expression vector containing the nucleic acid. When the reprogramming factor is introduced in the form of RNA, the immunogenic RNA introduced into the cell may activate the cell's defense mechanism, and thus RNA may be introduced into the somatic cell to circumvent this defense mechanism.

Examples of expression vectors include viral vectors such as retrovirus, lentivirus, adenovirus, adeno-associated virus, herpes virus, and Sendai virus, as well as plasmid vectors, episomal vectors, artificial chromosome vectors, and transposon vectors (piggyBac, piggyBat, TolII).

Nucleic acids, expression vectors containing the nucleic acids, or proteins (e.g., reprogramming factors) can be introduced into cells using various known methods. Such methods include, for example, calcium phosphate-mediated transfection, electroporation, liposome transfection, lipofection, gene gun, microinjection, viral vector method, virus-like particle method, Agrobacterium method, agroinfiltration method, PEG-calcium method, sonoporation method, lipid nanoparticle method, and the like.

### (Other Materials to be Supplied)

In addition to the above-mentioned substances, a sealed container storing a substance to be supplied to the processing container may be added as needed. The added sealed container can be connected to the processing container in the same manner as the other sealed containers. Other materials that may be supplied to the processing container include, for example, liquids, powders, additives, release agents, cryoprotectants, and CO₂.

In one embodiment, when the established induced pluripotent stem cells are intended to be subjected to expansion culture, examples of the additives include undifferentiated state maintenance factors. In this specification, the "undifferentiated state maintenance factor" refers to a substance that has the effect of suppressing differentiation of induced pluripotent stem cells, and is not particularly limited as long as it is such a substance. Examples of the undifferentiated state maintenance factors that are commonly used by those skilled in the art include bFGF, FGF2, FGF4, FGF8, EGF, Nodal, Activin A, Activin B, TGFβ1, and TGFβ2. When the additive is, for example, an undifferentiated state maintenance factor, the sealed container may be present as a third supply container as a component for carrying out expansion culture. The third supply container may be configured using all of the contents of the above-described first supply container and the later-described second supply container.

In another embodiment, when genome editing of established induced pluripotent stem cells is intended, a sealed container containing factors necessary for genome editing can be connected to the processing container. Examples of the genome editing include a method using zinc finger nucleases (ZFNs) that link a zinc finger DNA binding domain with a nonspecific DNA cleavage domain (Japanese Patent No. 4968498), a method using TALENs (TAL effector nucleases) that link a transcription activator-like (TAL) effector, which is a DNA binding module, with a DNA endonuclease (Japanese Unexamined Patent Publication No. 2013-513389), and a method using the CRISPR-Cas9 system that combines the DNA sequence CRISPR (Clustered Regularly Interspaced Short Palindromic Repeats) with the nuclease Cas protein family, which plays an important role together with CRISPR (Japanese Unexamined Patent Publication No. 2010-519929). For genome editing, a necessary factor can be, for example, an artificial nucleic acid cleaving enzyme whose nucleic acid binding domain includes a zinc finger, TALE, or PPR (Pentatricopeptide repeat). Furthermore, in the case of a CRISPR/Cas system, either class I or II may be used, and factors necessary for genome editing may include, for example, gRNA and Cas protein (including the Cascade complex). The Cas protein may be either wild-type or mutant. The factors necessary for genome editing may be in the form of nucleic acids, expression vectors containing nucleic acids, or proteins. Specifically, for example, when genome editing of artificial pluripotent stem cells is performed using the CRISPR-Cas9 system, genome editing can be performed by connecting, in a closed manner, a processing container to an electroporator, which is connected in a closed manner to a sealed container containing gRNA and Cas9 protein designed to edit a target sequence, and then introducing gRNA and Cas9 protein through electroporation.

### (Configuration for Producing Differentiated Cells)

In a preferred embodiment of the production device 10, as illustrated in FIGS. 1 to 3, the production device 10 may have one or more second supply containers 230 as a component for producing differentiated cells in situ from iPS cells produced in the processing container. The second supply container 230 is a sealed container for storing a fluid containing a substance for inducing differentiation of iPS cells in the processing container. Although the form of the sealed container may be any of the forms described in the description of the processing container, when the production device 10 is used as a closed system processing circuit portion that is attached to and detached from a commercially available cell processing device (such as CliniMACS Prodigy (registered trademark)), the sealed container (ii) made of the flexible material (a flexible bag used for cell culture bags, infusion bags, and the like) is preferable as the second supply container 230.

Like the first supply container, the second supply container 230 may be directly connected to the processing container 100 such that the material contained therein is fed to the inside of the processing container 100 while maintaining a closed system (FIG. 1), or may be connectable to the processing container via a unique connector C20 configured to prevent incorrect interconnection (FIG. 2). In either case, as shown in FIGS. 1 to 3, the second supply container 230 may be preferably connected to the processing container via a connecting conduit A10 that can be switched between a communication state and a non-communication state.

The second supply container 230 can be provided in the same number as the number of differentiation induction stages, and the processing container 100 may be provided with the same number of injection ports as the number of second supply containers 230, or with fewer injection ports by using a junction of connecting conduits and opening and closing valves.

### (Differentiated Cells)

In this specification, "differentiated cells" refers to cells or organoids obtained by inducing differentiation of induced pluripotent stem cells. Cells obtained may be undifferentiated cells such as stem cells and progenitor cells, or terminally differentiated cells. In this specification, a term "differentiated cell" may be used as a term that encompasses both an undifferentiated cell and a terminally differentiated cell obtained by inducing differentiation of induced pluripotent stem cells. In this specification, the term "undifferentiated cells" means cells that do not reach terminal differentiation in the cell lineage, and examples of the undifferentiated cells include stem cells other than pluripotent stem cells, and progenitor cells. Examples of stem cells or progenitor cells include ectodermal cells such as neural crest cells, neural stem cells, neural progenitor cells, glial progenitor cells, retinal stem cells, corneal stem cells, keratinocyte epidermal stem cells, melanocyte stem cells, and mammary stem cells, mesodermal cells such as hematopoietic progenitor cells, myeloid stem cells, lymphoid stem cells, B progenitor cells, T progenitor cells, mesenchymal stem cells, cardiac stem cells, cardiac progenitor cells, vascular endothelial progenitor cells, vascular pericytes, platelet progenitor cells, skeletal muscle stem cells, adipose stem cells, and kidney progenitor cells, and endodermal cells such as hepatic stem cells, liver progenitor cells, intestinal stem cells, and airway stem cells.

In this specification, "terminally differentiated cells" refers to cells that have reached terminal differentiation in a cell lineage. Examples of terminally differentiated cells include, but are not limited to, osteoblasts, chondrocytes, adipocytes, hepatocytes, hepatic mesothelial cells, bile duct epithelial cells, hepatic stellate cells, hepatic sinusoidal endothelial cells, Kupffer cells, pit cells, vascular endothelial cells, blood cells, pancreatic duct epithelial cells, pancreatic duct cells, centroacinar cells, acinar cells, islets of Langerhans, cardiac muscle cells, fibroblasts, smooth muscle cells, type I alveolar epithelial cells, type II alveolar epithelial cells, Clara cells, ciliated epithelial cells, basal cells, goblet cells, neuroendocrine cells, Kulchitsky cells, renal tubular epithelial cells, urothelial cells, columnar epithelial cells, glomerular visceral epithelial cells, glomerular endothelial cells, podocytes, mesangial cells, nerve cells, and glial cells. Examples of white blood cells include lymphocytes, granulocytes, and monocytes.

In an embodiment, the cells or organoids (target cells or organoids) obtained by inducing differentiation of induced pluripotent stem cells are neural crest cells, neural progenitor cells, nerve cells, cerebral cortical organoids, hematopoietic progenitor cells, platelets, T cells, or cardiac muscle cells. For example, T cells obtained by inducing differentiation of induced pluripotent stem cells can be modified into CAR-T cells (iCAR-T cells) using the method described in Nature Biomedical Engineering 5; 429:440; 2021.5.17, doi: 10.1038/s41551-022-00969-0. Furthermore, it is also possible to perform any desired genome editing on differentiated cells by additionally connecting an appropriate sealed container containing the necessary substances or a device such as an electroporator to the processing container.

### (iPS Cell Differentiation-Inducing Substance)

In this specification, the "differentiation-inducing substance" refers to a substance that can induce differentiation from induced pluripotent stem cells into the differentiated cells or organoids described above. The differentiation-inducing substance may be a substance known per se, or may be appropriately selected from substances that are commonly used for inducing differentiation of desired differentiated cells or organoids. Specifically, examples thereof include the substances described in "2. Cell Production Method According to the Present Invention" described below.

The fluid used as a medium for containing the reprogramming factors, undifferentiated maintenance factors, and differentiation-inducing substances is not particularly limited, but preferred examples thereof include buffer solutions, culture media, and cryoprotectants such as dimethyl sulfoxide (DMSO) and glycerin.

In the package according to the present invention, the liquid culture medium, solution, fluid, or the like stored in each sealed container may be cooled and frozen for the purpose of preservation during transportation or storage. In other words, liquid culture media, solutions, and fluids that are temporarily frozen in this manner fall under the category of "liquid culture media", "solutions", and "fluids" as used in this invention, as long as they remain liquid or fluid at the temperature at which they are used (when cells are processed).

### (Syringe Connection Port Provided on Sealed Container)

The sealed container has an injection port for dispensing, which may be, for example, a syringe connection port. A substance can be dispensed into the sealed container through the injection port using a syringe or pump.

### (Connecting Conduit)

The tubular member that constitutes the connecting conduit A10 is not particularly limited, and a tube (soft tube) made of a soft material (such as silicon or vinyl chloride) can be preferably used. The connecting conduit may partially include a pipe member made of a hard material. The sealed containers may be directly connected to each other via a connector, in which case the internal passage of the connector serves as the connecting conduit. When the production device 10 is used as a closed system processing circuit portion that can be attached to and detached from a commercially available cell processing device (such as CliniMACS Prodigy (registered trademark)), it is preferable to use a soft tube that is adapted to the pump (peristaltic pump) and pinch valve provided in the cell processing device.

### (Opening and Closing Valve)

In the present invention, a "connecting conduit which is switchable between a communication state and a non-communication state" is a connecting conduit whose flow path opens and closes in response to the operation of the opening and closing valve. Examples of the opening and closing valve include a clip that is installed on the connecting conduit A10 and manually openable and closable, and a pinch valve (an electromagnetic valve that presses and releases a soft tube from the outside) provided on a commercially available cell processing device (such as CliniMACS Prodigy (registered trademark)). The valve may be provided in advance as a component of the production device of the package, or may be provided as appropriate by a user who produces cells using the production device of the package at the time of use. In particular, when the production device is used as a closed system processing circuit portion that can be attached to and detached from a commercially available cell processing device (such as CliniMACS Prodigy (registered trademark)), it is preferable to use the opening and closing valve (such as the pinch valve) provided on the cell processing device.

### (Connector)

The connecting conduits may be connected to the respective sealed containers or to each other by direct joining, but preferably connected via an appropriate connector. Although connectors that can be used in the present invention are not particularly limited, examples thereof include luer connectors (such as old ISO594-1 and old ISO594-2), sterile connection joints, push-in joints for tubes, and one-touch joints. Sterile connection joints are preferred connectors that allow connection and disconnection while maintaining sterility within the pipe (i.e., within a closed system). The unique connector C20 configured to prevent incorrect interconnection is a sterile connector. As illustrated in FIG. 1, it is preferable that an appropriate connector C10 is provided at a leading end of each of the connecting conduits connected to the discharge port 102, the gas injection port 104, and the gas discharge port 105. More preferably, the appropriate connector C10 is sealed by heat sealing. Further preferably, the connecting conduit of the discharge port 102 is connected to a waste liquid bag.

In the present invention, the "unique connector configured to prevent incorrect interconnection" may be a sterile connector, for example, MicroCNX^{™} from Colder Products Company. Each pair of connectors may have different specifications from the other pairs of connectors. The unique connectors may be configured to alert the assembly worker to prevent incorrect connection, such as by applying a unique color to each pair of connectors. Particularly preferred are unique connector embodiments in which each pair of connectors is configured to be structurally incompatible with other pairs of connectors. These unique connectors provide an incorrect connection prevention effect as connectors that have been connected in advance. These unique connectors are also preferably sterile connection joints.

### (Feeding Device)

The feeding device is a device that moves substances (solutions, liquids, or fluids containing (somatic) cells) contained in each sealed container connected to the processing container to the processing container. The feeding device may be provided in advance as a component of the production device of the package, or may be provided as appropriate by a user who produces cells using the production device in the package at the time of use. Furthermore, as mentioned above, when the production device of the package is a closed system processing circuit portion that is attached to and detached from a cell processing device such as that shown in FIG. 16 (CliniMACS Prodigy available from MiltenyiBiotec, as well as others such as Gibco CTS Rotea Counterflow Centrifugation System available from Thermo Fisher Scientific, Cocoon system available from Lonza, and Xuri from Cytiva), the positions at which the containers (such as a culture medium container, and first and second supply containers) are hooked, the position of the feeding device (peristaltic pump), the position of the processing container, the path and length of the tubes, and the like differ depending on the manufacturer of such cell processing device, and the pinch valves that open and close also change depending on the operating program of the cell processing device. Therefore, it is preferable that the tube arrangement pattern of the production device according to the present invention is determined with reference to conventional piping conditions to be adapted to an intended cell processing device to which it is to be attached. There are no particular limitations on the feeding device that can be used in the present invention, and examples thereof include various conventionally known pumps and mechanisms that move using gravity. A power supply circuit, control circuit, connecting conduits, electromagnetic valves, and the like necessary for operating the feeding device may be added to the present invention as appropriate, or may be provided by the user as appropriate when using the production device. As will be described later, when the production device according to the present invention is a closed system portion of the cell processing device, the above-mentioned feeding device, power supply circuit, control circuit, connecting conduits, electromagnetic valves, and the like are all provided in the device body of the cell processing device.

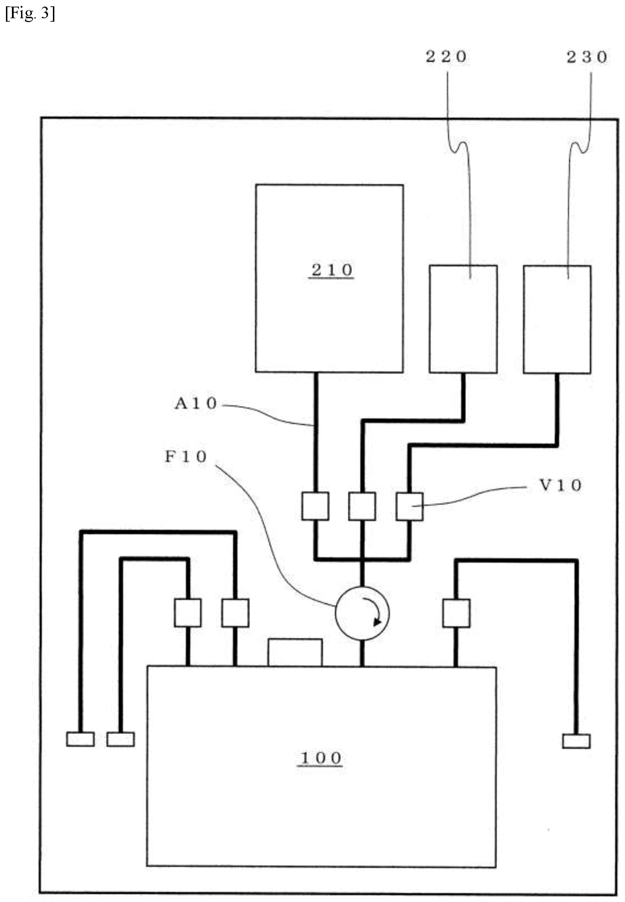

As illustrated in FIG. 3, a peristaltic pump F10 may be used to pump the contents of the culture medium container 210, the first supply container 220, and the second supply container 230 to the processing container (the peristaltic pump is represented by a symbol in FIG. 3). In order to use the peristaltic pump F10, a soft tube is used as the connecting conduit A10, and the soft tube may have a thickness, elasticity, and length that allows it to be attached to the peristaltic pump F10. Each connecting conduit is provided with an opening and closing valve V10, and the connecting conduit merges into a single connecting conduit, which is attached to the peristaltic pump F10 and can be connected to the processing container. By selectively opening the opening and closing valve V1, only the connecting conduit that passes through the selected opening and closing valve can feed substances. Therefore, by controlling three opening and closing valves V1 and a single peristaltic pump F10, only the substances in a desired sealed container can be fed to the processing container.

### (Relationship between Production Device of Package and Conventional Cell Processing Device)

In a preferred embodiment, the production device of the package does not have a feeding device, opening and closing valve, control device, power supply device, or the like, and is configured as a closed system portion (closed system processing circuit portion) that can be attached to and detached from the cell processing device such as that shown in FIG. 16. Examples of such cell processing devices include the CliniMACS Prodigy available from MiltenyiBiotec, the Gibco CTS Rotea Counterflow Centrifugation System available from Thermo Fisher Scientific, the Cocoon system available from Lonza, and the Xuri available from Cytiva. However, in addition to commercially available cell processing devices, cell processing devices created for the present invention (devices equipped with a feeding device, opening and closing valve, control device, power supply device, and the like such that the production device in the package can be used as a closed system portion and cells can be processed inside the closed system portion) may also be used. In such cell processing devices, the closed system portion is detachable from the mechanism portion (also referred to as the device body) while maintaining its own closedness. By providing the production device of the package according to the present invention in advance, or to a large extent, such that it can be attached as such a closed system portion, a user who wishes to product cells can simply attach the production device of the package to the cell processing device and start production of cells without the operation of connecting various sealed containers and preparing materials in a sterile manner, or with the amount of operation significantly reduced. As a result, the problem of incorrect connection on the user side is fundamentally resolved, or resolved to a large extent. All of the mechanism-related parts of the cell processing device (feeding device, opening and closing valve, control device (including a computer program for controlling cell culture), power source, air pressure source, and the like) can be used.

### (Sterilization of Cell Production Device)

The cell production device is filled with contents and connected to containers in a sterile manner. The cell production device in the package may be sterilized after being surrounded by the packaging material. Although the sterilization method is not particularly limited, examples thereof include radiation sterilization such as gamma ray sterilization.

### (Packaging Material)

The packaging of the package is preferably a packaging that surrounds the production device such that the sterilized state of the production device can be maintained. That is, the packaging is preferably a packaging such that bacteria, viruses, dust, foreign matter, and minute particles do not enter the package from the outside. The packaging material may not only be one that separates the inside from the outside, but also one that includes a gas-permeable membrane or porous filter (e.g., one with a pore size of about 0.2 µm or less, particularly about 0.1 to 0.2 µm). The packaging may enclose the production device in a liquidtight and airtight manner to seal the production device.

Although a specific form of the packaging is not particularly limited, examples of a preferred form thereof include the following:
A bag (flexible bag-like object) constituted by nonwoven fabric, paper, or flexible film;
A sealed container having a container body such as a tray made of a hard material and a flexible film that covers the opening of the container body;
A sealed container having a container body made of a hard material and a lid made of a hard material that covers the opening of the container body; and
A sealed container made of a composite material obtained by combining a component made of a flexible material and a component made of a hard material.

Although the material of the nonwoven fabric or flexible film used in the above-mentioned bag is not particularly limited, examples thereof include polymer materials such as polyethylene, polypropylene, nylon, polyester, polyvinyl chloride, polyvinylidene chloride, polymethylpentene, polyvinyl alcohol, ethylene-vinyl acetate copolymer, ethylene-vinyl alcohol copolymer, polycarbonate, polystyrene, polyacrylonitrile, and polyurethane, and a multilayer film made by combining these polymer materials as appropriate may also be used. The flexible film may be a gas permeable film. Materials other than the above polymer materials, such as a laminate layer made of an aluminum film or an aluminum vapor deposition layer, may also be used in appropriate combination.

### (Structure or Method for Closing Opening of Bag)

A structure or method for closing the opening of the bag is not particularly limited, and may be one that is repeatedly openable and closable, or one that requires destruction or cutting to open, and examples thereof include known fasteners (zippers), sealing by heat welding, sealing with an adhesive, and sealing with a clip, sealing tape, or the like.

Although the hard material is not particularly limited, examples thereof include polymer materials that have traditionally been used as materials for hard containers, such as polyethylene, polyethylene terephthalate, polypropylene, polyester, polycarbonate, polystyrene, polyvinyl chloride, acrylonitrile-styrene, polymethylpentene, polymethyl methacrylate, acrylonitrile-butadiene-styrene, polyacetal, and polyamide, as well as metals such as stainless steel and aluminum alloys.

The above-mentioned packaging is preferably further surrounded and packaged in a packaging material with higher mechanical strength (such as a bag or box made of a thicker material) for the purpose of protecting the device from dirt and the like during transportation and storage. Examples of the materials include styrofoam, cardboard, and plastic cardboard. The additional packaging is preferably the same packaging box when it is received and when it is disposed of. When the packaging box used when the package is received is the same as the packaging box used when it is disposed of, the received package will be stored in the box it was stored in when it was received and then disposed of, which makes it easy to trace the disposal and is also preferable from the perspective of environmental protection.

It is preferable that the packaging made of a packaging material further satisfies one or more of the following conditions:
- Even when the production device is subjected to vibrations or the like or tilted, the production device is fixed in the internal space surrounded by the packaging material such that each portion of the production device does not move, and it can be positioned such that it does not shift or get damaged due to vibrations or the like. For example, a flexible bag may be used as the packaging material, the inside of which is decompressed as in vacuum packaging, and flexible films may be fixed in an intimate contact with each portion of the production device to fix the portions of the production device.
- It is preferable that the packaging material does not have any unintended openings such as pinholes, and that there is no internal or external circulation.
- It is preferable to follow sterile medical device packaging guidelines.
- It is preferable that sterilization validation is carried out during sterilization.
- Packaging validation is carried out during packaging. It is preferable that conformity with sterilization means, stability during transportation or storage (such as vibration, temperature change, and light-shielding performance), and the like are taken into consideration.

### (Specific Examples of Cell Processing Device, Device Body, Closed System Portion, and Package)

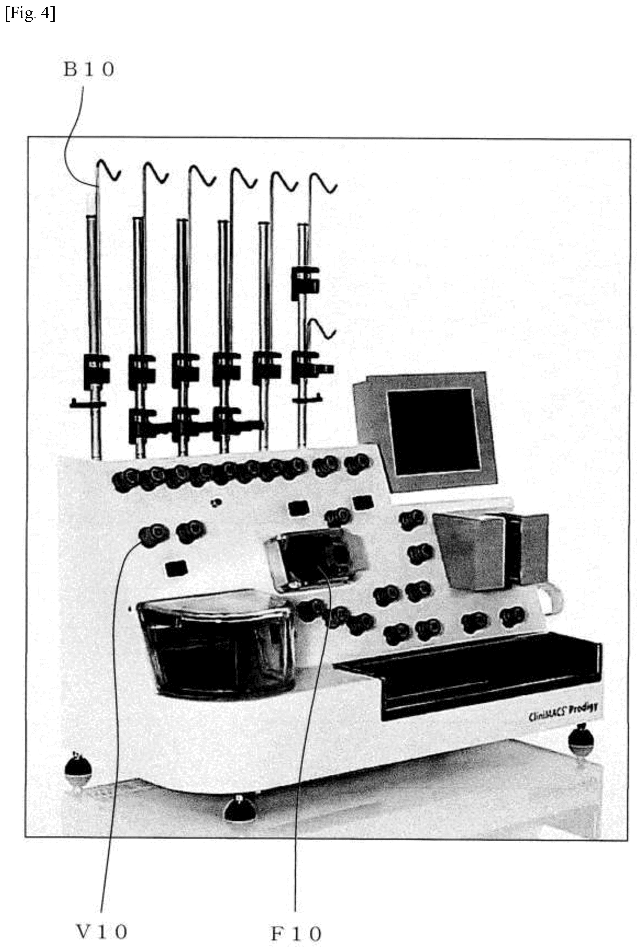

FIG. 4 is a photograph showing a device body of a commercially available automatic cell processing device, CliniMACS Prodigy (also referred to as "Prodigy" hereinafter) available from Miltenyi Biotec, from which the closed system portion (the cell production device in the present invention) has been removed. As shown in FIG. 4, the device body of the Prodigy has a support B10 for suspending a bag such as a culture medium container or a first supply container, and also has necessary actuators such as multiple pinch valves V10 that open and close according to a predetermined program, and a peristaltic pump F10 that moves a fluid inside the closed system portion.

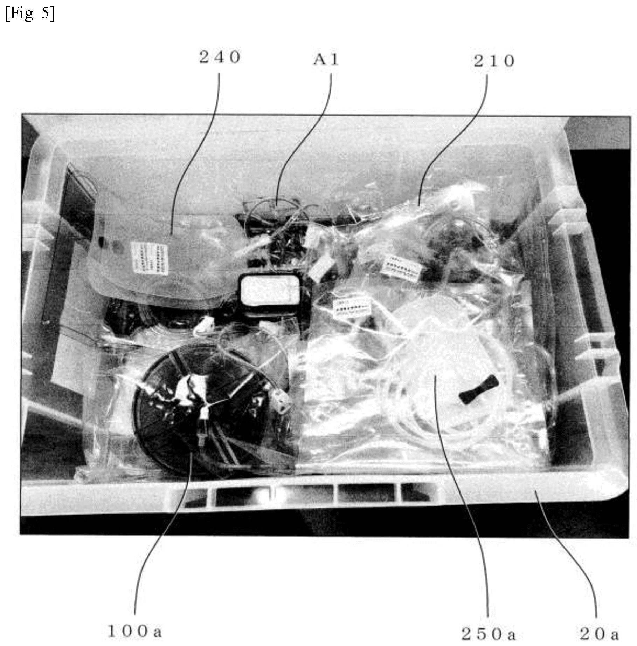

FIG. 5 is a photograph showing the inside of the package accommodating the closed system portion to be attached to the device body of Prodigy shown in FIG. 4. As shown in FIG. 5, a processing container 100a dedicated to Prodigy, a culture medium container 210, a connecting conduit section A1 configured to fit the layout of the device body, a sealed container 240 used for processing, a blood collection device 250a, and the like are housed inside a packaging material (packaging container) 20a. Also, the closed system portion may be connected to an empty sealed container for storing target cells to be produced. These elements are either assembled as a closed system portion or are available for assembly via unique connectors. The blood collection device 250a is sent to a medical institution and used to collect blood from a patient. After blood collection, the blood collection device 250a is given a code necessary for identification, such as a patient identification number or a cell production identification code, and is sent to the cell producer. In the example of FIG. 5, the blood collection device 250a is included in the package, but it may be packaged separately from the package for delivery to a medical institution.

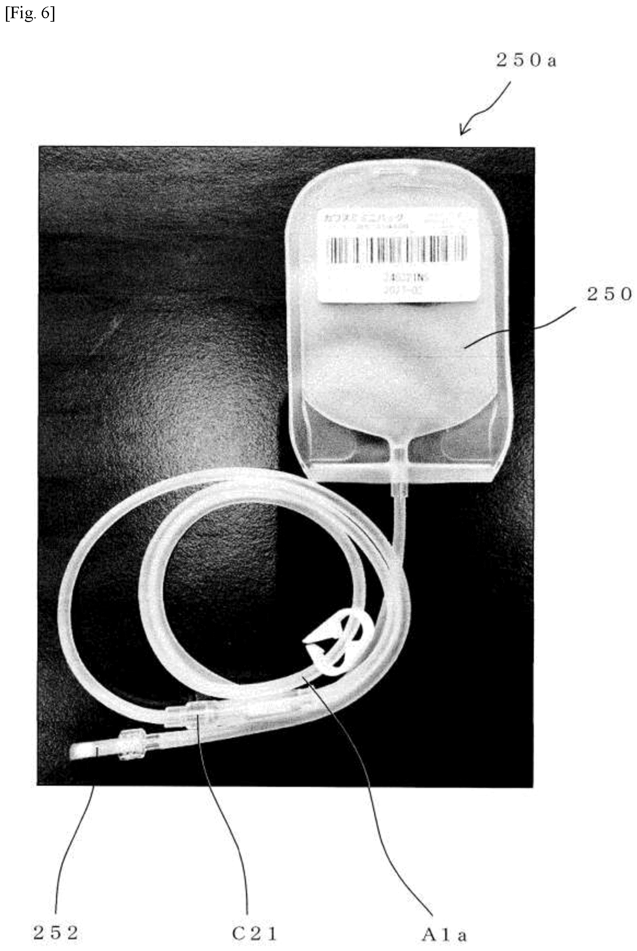

FIG. 6 is a photograph showing an example of the blood collection device 250a. The blood collection device 250a has a small blood collection bag 250 and a conduit (tube) A1a connected thereto. A butterfly needle (or a general blood collection needle) used for blood collection is connected to a leading end of the conduit A1a, and a unique connector is inserted into an intermediate portion of the conduit A1a for aseptic connection to a predetermined position of the closed system portion.

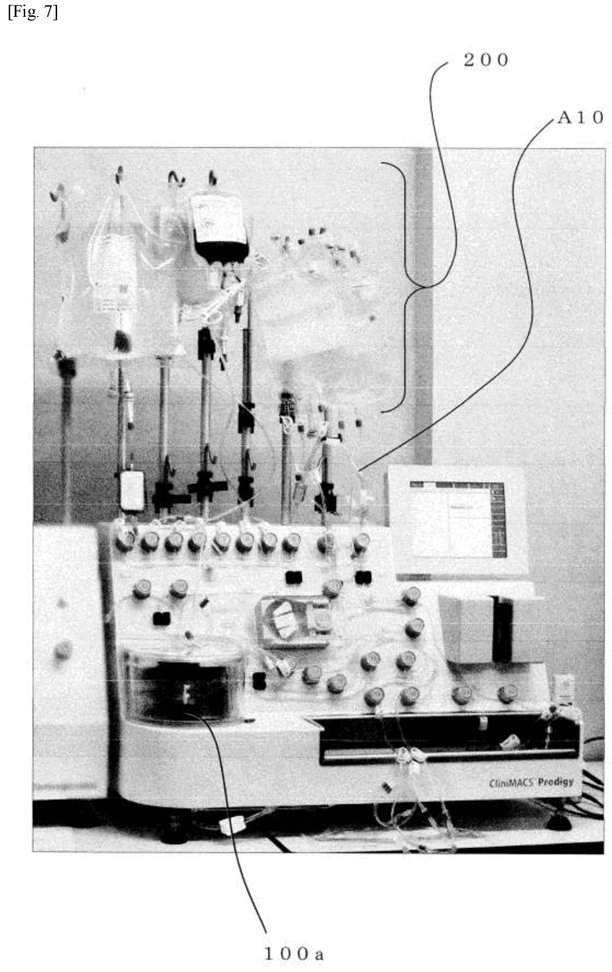

FIG. 7 is a photograph illustrating a state in which the closed system portion is attached to the device body of the Prodigy shown in FIG. 5. The various sealed containers (bags) 200 included in the closed system portion are suspended from the support B10 shown in FIG. 5, a soft tube A1, which is a connecting conduit, is set to pass through a predetermined pinch valve and peristaltic pump, the processing container 100a is set on a drive device to enable rotation for centrifugation, and a computer program for cell processing is ready to start.

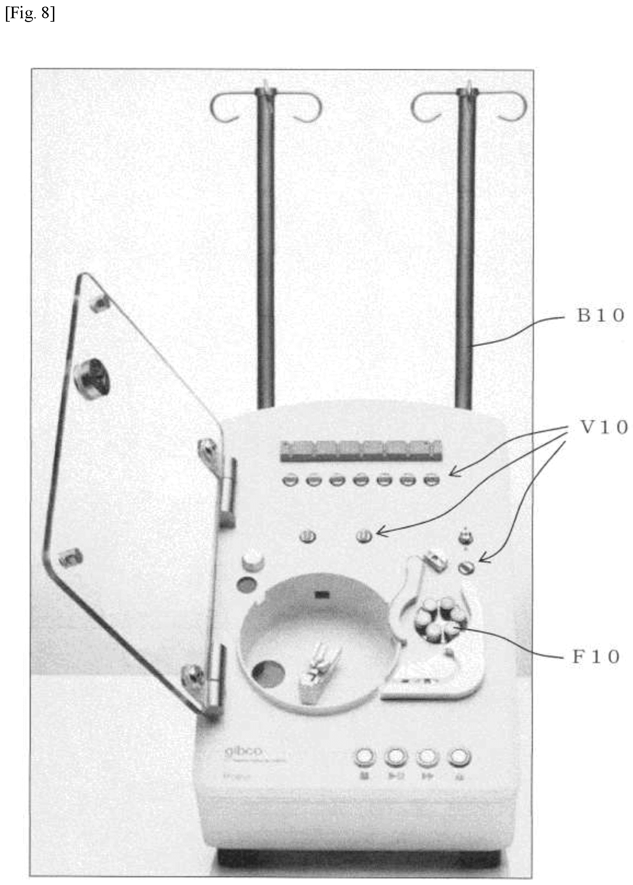

FIG. 8 is a photograph showing the device body of a commercially available automatic cell processing device, the Gibco CTS Rotea Counterflow Centrifugation System (also referred to as "Rotea" hereinafter) from Thermo Fisher Scientific, from which the closed system portion has been removed. As shown in FIG. 8, the device body of the Rotea has a support B10 for suspending a bag such as a culture medium container or a first supply container, and also has necessary actuators such as multiple pinch valves V10 that open and close according to a predetermined program, and a peristaltic pump F10 that moves a fluid inside the closed system portion.

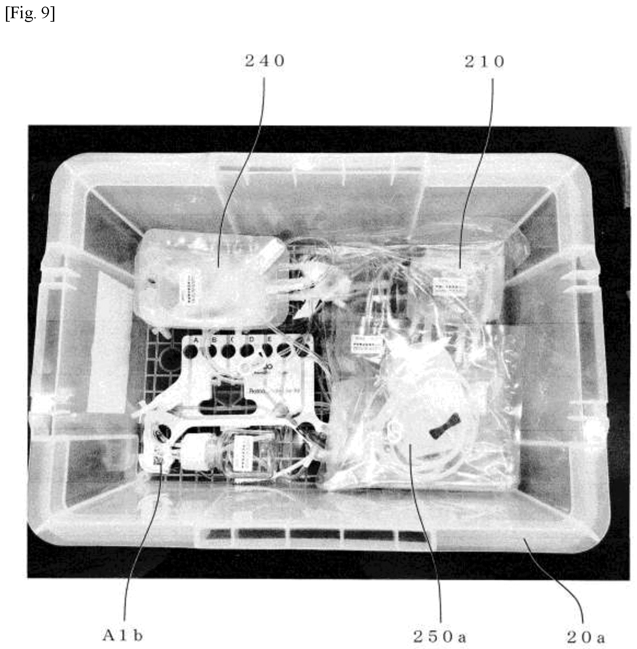

FIG. 9 is a photograph showing the inside of the package accommodating the closed system portion to be attached to the device body of Rotea shown in FIG. 8. As shown in FIG. 9, a unit A1b containing a dedicated processing container for Rotea, the culture medium container 210, the connecting conduit (hidden below the unit A1b) that follows the layout of the device body of the Rotea, the sealed container 240 used for processing, the blood collection device 250a, and the like are contained inside the packaging material (packaging container) 20a. As in the example shown in FIG. 5, these elements are assembled as the closed system portion or are available for assembly via unique connectors. The blood collection device 250a is also similar to the example shown in FIG. 5.

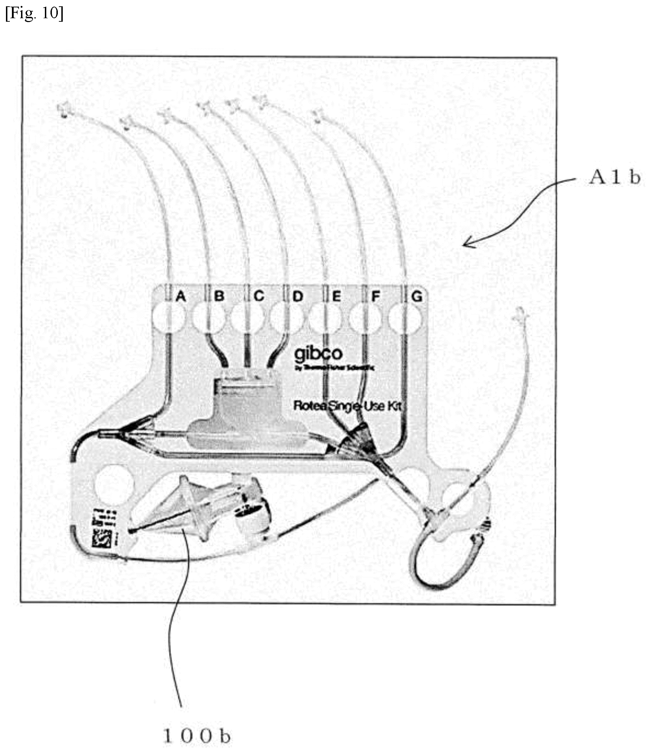

FIG. 10 is a photograph showing the unit A1b shown in FIG. 9. The unit A1b is a dedicated disposable component called a single-use kit that engages with the device body of the Rotea, and includes a conical processing container (centrifugation chamber) 100b for performing counterflow centrifugation.

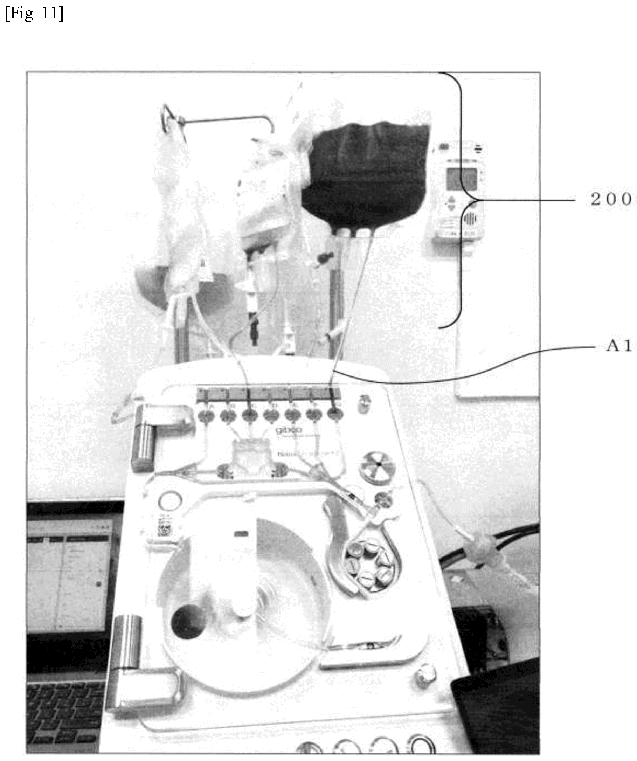

FIG. 11 is a photograph illustrating a state in which the closed system portion is attached to the device body of the Rotea shown in FIG. 8. Various sealed containers (bags) 200 included in the closed system portion are suspended from the supports B10 shown in FIG. 8, and a connecting conduit (soft tube) A1 extending from each sealed container is connected to the unit A1b. When the computer program for cell processing starts, each connecting conduit opens and closes with a predetermined pinch valve. Also, the connecting conduits converge into one pipe that passes through a peristaltic pump. The processing container 100b is set in a drive device such that it can be rotated for centrifugation. In the example shown in FIG. 11, the device body has started a cell processing operation, the processing container 100b is rotating, and fluid is moving inside the closed system portion.

As specifically illustrated in FIGS. 4 to 11, the cell production device packaged in the packaging material of the package is a closed system portion included in various cell processing devices. That is, each cell processing device is configured to include the device body and the closed system portion.

As illustrated in FIGS. 4 and 8, each device body has an operating mechanism for operating the closed system portion and a control unit that controls the operating mechanism. The closed system portion is attachable to and detachable from the device body while maintaining the airtightness of the closed system itself. In the present invention, the closed system portion is assembled in a state where it is detached from the device body, and is provided to the cell producer in a form that is adapted to and can be attached to each device body. The closed system portion attached along a predetermined position of the device body is actuated by receiving an external force from the operating mechanism. The operating mechanism operates under the control of the control unit of the device body, and the control unit operates the operating mechanism in accordance with a predetermined operating program. As a result, somatic cells are transferred and processed within the closed system portion to produce target cells.

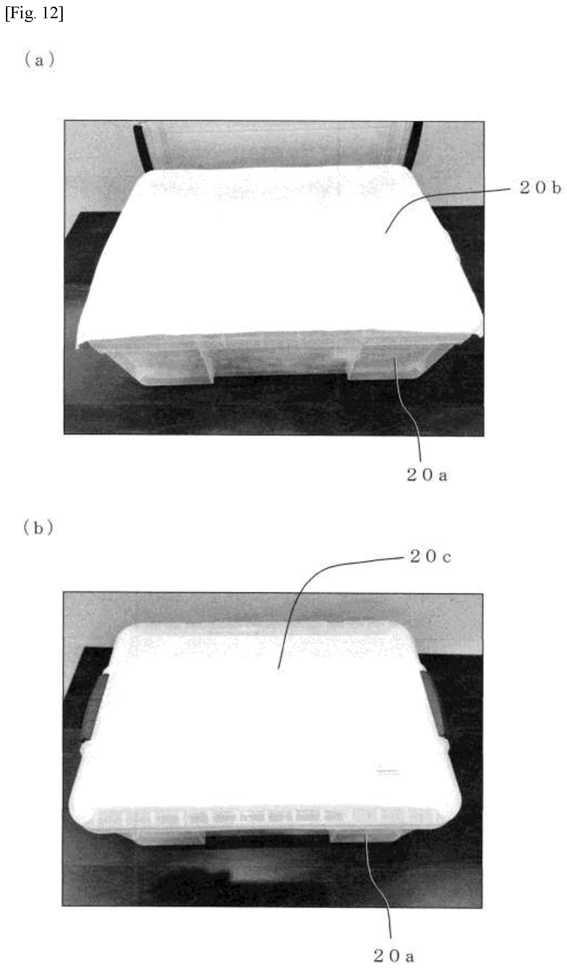

FIG. 12 is a photograph showing an example of a configuration of the packaging material of the package. In the example shown in FIG. 12, the opening of the container body 20a made of a hard resin material is sealed with a flexible film (inner lid) 20b (FIG. 12(a)), and further, an outer lid 20c is fixed to the container body 20a, covering the flexible film 20b. In the example shown in FIG. 12, the flexible film 20b is made of a high-density polyethylene nonwoven fabric (Tyvek (registered trademark)), which allows permeation of gas while preventing viruses and the like from entering from the outside. Also, an outer lid 20c is made of a hard resin material, similar to the container body 20a. This double-lid structure keeps the inside of the package sterile, while also making it possible to withstand external forces and stack the packages.

### (Other Preferred Embodiments of Package according to the Present Invention)

The package may be in the following form:
(a1) A package of a cell production device comprising:
   a cell production device; and a packaging material;
   wherein the cell production device includes
      a processing container, and
      one or more fluid supply containers for storing fluids used in processing the cells,
   the fluid supply containers are, such that their contents are fed into the processing container while maintaining a closed system,
      (i) connected to the processing container via a connecting conduit that can be switched between a communication state and a non-communication state, or
      (ii) connectable to the processing container via the connecting conduit and via a unique connector configured to prevent incorrect interconnection,
   the processing container has at least an injection port that is openable and closable for receiving a solution containing somatic cells from an outside, and a discharge port that is openable and closable for discharging the contents,
   the processing container and the fluid supply containers are sealed containers, and
   the cell production device is packaged in the packaging material.
(a2) The package of a cell production device according to (a1) above, wherein the cell production device is a detachable closed system processing circuit portion in a cell processing device including the detachable closed system processing circuit portion, a mechanism that operates the processing circuit portion, and a control unit.
(a3) The package of a cell production device according to (a1) or (a2) above, wherein the one or more fluid supply containers include at least one culture medium container for storing a liquid culture medium.
(a4) The package of a cell production device according to any one of (a1) to (a3) above, wherein the one or more fluid supply containers include a first supply container for storing a fluid containing the reprogramming factor as a configuration for producing induced pluripotent stem cells.
(a5) The package of a cell production device according to any one of (a1) to (a4) above, wherein the one or more fluid supply containers include a third supply container for storing a fluid containing an undifferentiated state maintenance factor as a configuration for carrying out expansion culture of the established iPS cells.
(a6) The package of a cell production device according to any one of (a1) to (a5) above, wherein the one or more fluid supply containers includes one or more second supply containers for storing a fluid containing a differentiation-inducing substance for the induced pluripotent stem cells as a configuration for producing differentiated cells from the induced pluripotent stem cells produced in the processing container.

The "fluid supply container" in (a1) above may be configured in the same manner as in the above-described first supply container and second supply container.

In the above embodiments (a1) to (a5), for example, the package of the present invention can be used to perform the following processes:
A process in which using a package (first package) of a cell production device configured to establish iPS cells, the cell production device in the first package is attached to the Rotea, which is a known cell processing device, thereby separating white blood cells from whole blood and further establishing iPS cells;
A process in which using a package (second package) of a cell production device configured to carry out expansion culture of the established iPS cells, the cell production device in the second package is attached to the Cocoon, which is a known cell processing device, thereby subjecting the established iPS cells to expansion culture; and
A process in which using a package (third package) of a cell production device configured to produce differentiated cells from iPS cells, the cell production device in the third package is attached to the Prodigy, which is a known cell processing device, thereby producing differentiated cells from iPS cells.

### (Preferred Examples of Use of Package according to the Present Invention)

In terms of convenience for cell producers, the package according to the present invention is preferably in a form in which a completed single closed system portion is housed in a single package. However, in addition to such a single package form, the package may be provided to the cell producer in a separated form in two or more packages depending on the request of the cell producer and various circumstances such as management issues regarding the elements. That is, the package may be in a form such that a predetermined element contained in one closed system portion is separated from the closed system portion and packaged in a different packaging material. For example, one or both of the culture medium container and the first supply container that constitute the closed system portion may be separated from the closed system portion and packaged in different packaging materials, resulting in the package being separated into three packages that are independent of each other. Even when the package is in the form of two or more separated packages, the contents of the packages are properly assembled into one closed system portion via a unique connector by the cell producer. When contents such as reagents are stored in the sealed containers (such as the culture medium container, the first supply container, and the second supply container) included in the closed system portion and the connections are complete, no operation prior to cell production is required and the cell production operation becomes more efficient. Thus, it is preferable that the closed system portion includes the first supply container, and it is even more preferable that the closed system portion also includes a culture medium container.

One of the problems in managing the above-mentioned elements is that the elements have different management temperatures. In other words, the contents of the sealed containers in the cell production device in the package may include a content that can be stored or distributed at room temperature (e.g., about 1°C to 30°C) or ambient temperature (e.g., about 15°C to 25°C), as well as a content that requires temperature control such as cooling (e.g., about 2°C to 8°C) or freezing (e.g., about -20°C to -196°C) during storage or distribution. In such cases, a package A containing an item that can be stored or distributed at room temperature or ambient temperature, and a package B containing an item that requires temperature control such as cooling or freezing can be produced (when there are multiple temperatures to be controlled, multiple packages B, C, D, and ... may be produced for each temperature), the package A may be provided to the user at ambient temperature, and packages that require temperature control (package B and the like) may be provided to the user in a cooled or frozen state, and the cell production device and sealed containers that are stored in these packages may be assembled by the user into a single cell processing device (such as the Prodigy mentioned above) to form a single closed system processing circuit portion.

More specifically, liquid culture media may need to be refrigerated at about 4°C during transportation and storage, and viral vectors (such as SeV) for introducing reprogramming factors into somatic cells may need to be frozen at about -80°C during transportation and storage. Therefore, most of the closed system portion may be provided to the user as a package A at ambient temperature, a package B containing the culture medium container may be provided to the user at a required refrigeration temperature, and a package C containing the first supply container may be provided to the user at a required freezing temperature. Also, the culture medium container may be included in most of the closed system portion and may be provided to the user as the package A at the required refrigerated temperature, and the package B containing the first supply container may be provided to the user at a required freezing temperature. A packaging material may be configured including an ambient temperature region, a refrigeratable region, and a freezable region, and each element may be appropriately arranged in these areas.

The culture medium container included in the closed system portion may be an empty container for storing a liquid culture medium, and the cell producer may pour the liquid culture medium into the empty container. The first supply container included in the closed system portion may also be an empty container for storing a fluid containing the reprogramming factor, and the cell producer may perform a process for, for example, thawing the reprogramming factor stored in the cell production facility, mixing the reprogramming factor with a liquid culture medium in a safety cabinet, and filling the container with the mixture, or filling a container with the reprogramming factor via a sterile connector, and then inject the fluid containing the reprogramming factor into the empty container.

The cell production step of processing somatic cells (including blood) collected from a patient to produce target cells is preferably started within about 0 to 24 hours from the time the somatic cells are collected from the patient. Therefore, it is preferable that the start date and time of the cell producing process and the date and time of collection of the somatic cells, which serve as a raw material, are determined in relation to each other.

The cell production step takes a long period of time, for example, one month or the like, and therefore, in addition to materials necessary on the first day of production, predetermined materials may be necessary for each elapsed time or day. Therefore, in response to such requests, it is preferable to provide additional packages, following the first provided package (containing all the materials necessary at the start of cell production), in which sealed containers (such as bags) containing the predetermined materials necessary for each elapsed time or day, such as the second day, the third day, through the 28th day, from the start of production. Examples of such additionally provided materials include bags, connecting conduits, reagents, culture media, and the like.

### 2. Cell Production Method according to the Present Invention

### (Method for Producing iPS Cells)

Next, a cell production method according to the present invention (also referred to as the "production method" hereinafter) will be described with reference to the production device illustrated in FIGS. 1 to 3.

The production method is a cell production method using the package according to the present invention described above. The production method includes a step of preparing a production device taken out from the package, and a step of producing at least iPS cells inside a processing container 100 of the production device 10 shown in FIG. 1. The elements constituting the production device 10 and the substances stored in the sealed containers are as described above. The production device 10 is equipped with a feeding device, a valve, and a control device as needed, and the feeding device and the valve are operated to feed materials from the sealed containers to the processing container, thereby carrying out the process of producing iPS cells.

The step of producing iPS cells includes at least the following steps:
Step (s10): Injecting a solution containing somatic cells into the processing container 100 through the injection port 101, feeding a liquid culture medium from the culture medium container 210, and feeding a fluid containing a reprogramming factor from the first supply container 220, thereby bringing the reprogramming factor into contact with the somatic cells in the liquid culture medium within the processing container; and
Step (s20): Culturing the somatic cells in the liquid culture medium in the processing container 100 to establish iPS cells.

### (When Processing Container Has Function of Separating Somatic Cells)

When the processing container 100 has the function of separating somatic cells, in Step (s10), a raw material such as the above-mentioned whole blood or urine is directly injected into the processing container through the injection port 101, as illustrated in FIG. 1. Depending on the function of separating somatic cells, substances necessary for the separation are further injected into the processing container 100, and somatic cells for use for producing iPS cells are separated within the processing container, and components other than the somatic cells are discharged to the outside of the processing container, leaving the somatic cells to be processed inside the processing container. Then, the liquid culture medium is fed from the culture medium container 210, and the fluid containing a reprogramming factor is fed from the first supply container 220, thereby bringing the reprogramming factor into contact with the somatic cells in the liquid culture medium within the processing container 100. Thereafter, iPS cells are established in the Step (s20) mentioned above.

### (Expansion Culture and Quality Evaluation after iPS Cell Establishment)

The induced pluripotent stem cells established by the production method according to the present invention may be subjected to expansion culture as appropriate using a known method. After expansion culture, the quality of the obtained iPS cells may be evaluated using a known method.

### (Method for Producing Differentiated Cells)

In a preferred embodiment, the production method further includes the Step (s30) of forming differentiated cells in the processing container 100 after the Step (s20). In order to carry out the Step (s30), the production device having a second supply container 230 is used. After the Step (s20), a fluid containing an iPS cell differentiation-inducing substance (a substance for inducing differentiation of iPS cells) is supplied from the second supply container 230 into the processing container 100, and differentiated cells are formed within the processing container. The differentiated cells to be produced and the substances to be supplied for inducing differentiation are as described above. If the differentiation induction needs to be carried out in multiple stages to obtain cells to be produced, second supply containers 230 can be provided in the same number as the types of substances necessary for each differentiation induction, and the substances can be controlled to be fed to the processing containers in the order of the differentiation induction stages.

In the cell production method according to the present invention, a known method can be used as a differentiation induction method to obtain target cells or organoids. For example, differentiation of pluripotent stem cells into neural crest cells can be induced using methods such as those described in Fukuta M. et al., PLoS One, 2014, 9(12): e112291 and Kamiya D, et al., NPJ Regen Med., 2022 Sep 15;7(1):47, and the like. Specifically, pluripotent stem cells can be differentiated into neural crest cells by seeding them into a culture vessel and culturing them in adherent culture (floating culture using a scaffold material), and then culturing them in adherent culture (floating culture using a scaffold material) in a culture medium containing a TGFβ inhibitor and a GSK3β inhibitor.

It is also possible to produce, from neural crest cells, cells such as mesenchymal stem cells, neural progenitor cells, nerve cells, glial cells, bone cells, chondrocytes, corneal cells, and melanocytes. For example, differentiation into these cells can be induced based on methods such as those described in Fukuta M. et al., PLoS One, 2014, 9(12): e112291, Horikiri T. et al., PLoS One, 2017, 12(1): e0170342, and Kamiya D, et al., NPJ Regen Med., 2022 Sep 15;7(1):47, and the like. Specifically, for example, neural progenitor cells and nerve cells can be obtained by seeding neural crest cells onto a fibronectin-coated plate, replacing the culture medium with DMEM/F12 supplemented with N-2 Supplement, BDNF, GDNF, NT-3, and NGF, and culturing the cells at 37°C under 5% CO₂ for about 14 days. Alternatively, neural progenitor cells and nerve cells can be obtained by seeding neural crest cells onto a plate and culturing the cells in the CDM containing SB431542 and CHIR99021 for one day, then replacing the medium with Neurobasal medium containing B-27 Supplement, N-2 Supplement, L-glutamine, Penicillin/Streptomycin, BDNF, GDNF, NT-3, and NGF, and culturing the cells at 37°C under 5% CO₂ for about 35 days.

The differentiation of cells into mesenchymal stromal cells can be induced, for example, using the following method. Neural crest cells are seeded in a culture vessel and cultured for 1 day in the CDM medium containing SB431542 and CHIR99021. After one day, the medium is replaced with αMEM containing FBS. About 14 days after the start of differentiation induction, mesenchymal stromal cells can be obtained.

Examples of a method for differentiating pluripotent stem cells into T cells include a method including (1) a step of differentiating pluripotent stem cells into hematopoietic progenitor cells and (2) a step of differentiating the hematopoietic progenitor cells into T cells. The step (1) may be a step of culturing pluripotent stem cells in a culture medium for inducing hematopoietic progenitor cells, as described, for example, in WO2013/075222, WO2016/076415, Liu S. et al., Cytotherapy, 17 (2015); 344-358, and the like. The step (2) may be (2-1) a step of inducing CD4CD8 double-positive T cells from hematopoietic progenitor cells, or (2-2) a step of inducing CD8 positive T cells from CD4CD8 double-positive T cells, as described in WO2016/076415 and the like.

Examples of the step of inducing differentiation of pluripotent stem cells into platelets include a method including (1) a step of differentiating pluripotent stem cells into hematopoietic progenitor cells, and (2) a step of differentiating hematopoietic progenitor cells into platelets. The step (2) may be a step of culturing hematopoietic progenitor cells in a culture medium containing TPO and/or SCF for about 7 to 15 days, as described, for example, in WO2012/157586, US2014/127815, Nakamura, Eto, et al. Cell Stem Cell 14, 535-548 (2014), and the like. This step yields a cell population containing megakaryocytes and platelets.

Examples of a method for inducing differentiation of pluripotent stem cells into cardiomyocytes include the methods described in WO2015/141827 and Laflamme MA and Murry CE, Nature. 473(7347):326-35 (2011). Other methods include, for example, a method of producing cardiomyocytes by forming embryoid bodies from induced pluripotent stem cells through floating culture, a method of producing cardiomyocytes in the presence of a substance that suppresses BMP signaling (WO2005/033298), a method of producing cardiomyocytes by sequentially adding Activin A and BMP (WO2007/002136), and a method of producing cardiomyocytes in the presence of a substance that promotes activation of the canonical (classical) Wnt signaling pathway (WO2007/126077). Typically, examples of marker proteins for cardiomyocytes include NKX2.5 (a cardiac muscle-specific transcription factor) and TNNT2 (troponin T), while examples of marker proteins for cardiac progenitor cells include KDR (a receptor for vascular endothelial growth factor (VEGF)) and ISL1 (a LIM homeodomain transcription factor).

Organoids can also be produced using multiple types of cells. For example, in the case of hepatic organoids, as described in WO2013/047639 or the like, hepatic organoids can be produced by inducing hepatic progenitor cells (organ cells), mesenchymal stem cells, and vascular endothelial cells from pluripotent stem cells, and subjecting the mixture thereof to floating culture.

In the cell production method of the present invention, the culture may be conducted under feeder-free conditions and/or xeno-free conditions over the entire period of the culture or during a part of the period of the culture. From the viewpoint of a clinical application, it is preferable to conduct the differentiation induction method of the present invention under feeder-free conditions and xeno-free conditions throughout the entire period.

The cell production method of the present invention may include a step of collecting obtained cells or organoids of interest. The collected cells may be cryopreserved using a cell cryopreservation solution. The number of cells collected in a container may be counted using a cell counter, and the cells may be labeled with an antibody against cell surface marker and then purified using flow cytometry, mass cytometry, a magnetic cell sorting method, or the like.

In the cell production method according to the present invention, undifferentiated cells may be removed as appropriate. The method for removing undifferentiated cells is not particularly limited as long as it can remove cells other than cells produced using the cell production method according to the present invention, and the undifferentiated cells can be removed by adding a known undifferentiated cell removing agent or the like to the culture medium (e.g., Di Mao., et al. Angewandte Chemie International Edition; 9 January 2017; Ben-David, U., et al. Cell Stem Cell, 12, 167 (2013); WO2019/187918; Japanese Unexamined Patent Publication No. 2016-93178; Yoshiki Nakashima, et al., Molecular Therapy Vol. 26 No. 7 July 2018, and the like).

Quality testing may also be carried out as appropriate to determine whether the cells, organoids, or the like obtained by the cell production method of the present invention are desired. The test items in the quality testing are not particularly limited, but include basic tests such as the morphology of cells and organoids, the presence or absence of cell surface marker expression, sterility tests, endotoxin tests, and evaluation of cell viability, and various testing devices are available for each item.

With regard to other matters necessary for the cell production method according to the present invention, all of the contents described above in "1. Package of the cell production device" are incorporated herein by reference.

### 3. Management System according to the Present Invention

Next, a management system according to the present invention (also referred to as the "management system" hereinafter) will be described.

The management system is for managing the step of producing the package according to the present invention described above, and in a preferred embodiment, is a system for detecting whether the package meets specifications when cells are produced using the package.

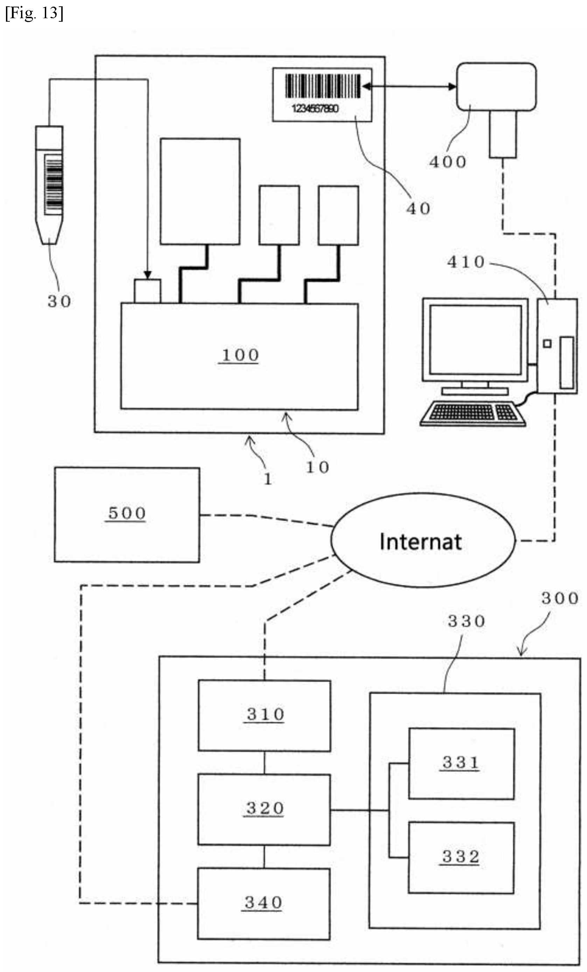

As shown in an example of the configuration in FIG. 13, the management system includes a management device 300 and a reading device 400 for reading the identification code. In the example shown in FIG. 13, the management device 300 is a server computer (also called a Web server) located on the Internet, and the reading device 400 may be connected to a terminal computer 410 to enable data communication, and the terminal computer 410 may be connected to the management device 300 to enable data communication via the Internet. In a preferred embodiment, the terminal computer 410 is a tablet terminal, and the reading device 400 is a camera mounted on the tablet terminal. The terminal computer 410 may also be a data input device for inputting the specification data described below. The management device 300 may be a terminal computer capable of data communication with other terminal computers.

In the management system according to the present invention, an identification code (D1) assigned to each package is displayed to be readable by the reading device 400 for all of the various packages produced. The identification code (D1) may be displayed on one or both of the packaging material and the cell production device. In the example of FIG. 13, as a preferred embodiment, the identification code (D1) is displayed on the packaging material of the package in the form of a barcode 40 (in the example shown in FIG. 13, the numbers of the identification code are also written). The identification code may be displayed in the form of a two-dimensional symbol (two-dimensional code). Hereinafter, the display of an identification code in the form of a symbol that can be read by the reading device (in the form of an information carrier such as a barcode, a two-dimensional symbol, or an IC tag) will also be simply referred to as "the identification code being displayed".

In the present invention, an identification code is a combination of numbers, letters, symbols, figures, and the like that is assigned to each object to be identified so as to be unique.

In the present invention, the wording "the identification code is displayed to be readable by a reading device" means that, depending on the reading function of the reading device, the identification code is displayed on the object as a combination of numbers, letters, symbols, figures, and the like, or in the form of a one-dimensional symbol (such as a barcode (also called a one-dimensional code)), or in the form of a two-dimensional symbol (such as a data matrix or QR code (registered trademark) (also called a two-dimensional code)), or in the form of any other symbol, or in the form of an IC tag. For example, when the reading device is a device that reads one-dimensional symbols, the identification code is accordingly displayed on the surface of the object in the form of a one-dimensional symbol. Attaching an IC tag also corresponds to the display of an identification code. The IC tag may be located inside the object as long as it can be read by the reading device. The reading device may read the identification code by using a character recognition, image recognition, or voice recognition function. The identification code in the form of a one-dimensional or two-dimensional symbol may be printed directly on the surface of the object, or a sheet on which the identification code is printed may be attached to the surface of the object. For the technology of the identification code and the reading device, a conventionally known technology can be referred to. When an identification code contains a large amount of information, such as a two-dimensional symbol or an IC tag, the identification code may be the identification information itself to be associated with the identification code.

As shown in FIG. 13, the management device 300 has a data receiving unit 310 and an attribute information storage unit 331. The data receiving unit 310 receives the identification code read by the reading device 400 as input data. The attribute information storage unit 331 stores the respective identification codes (D1) of all the packages to be produced and respective pieces of product attribute information (P1) of all the packages in a mutually associated state. The management device 300 is configured to refer to the identification code (D1) received by the data receiving unit 310 as input data, and the identification code (D1) and the product attribute information (P1) stored in the attribute information storage unit 331, and identify and output the product attribute information (P1) associated with the read identification code (D1). As shown in FIG. 13, the attribute information storage unit 331 may be provided in a storage device 330. The storage device may be implemented inside the management device 300 or may be an external device, and may be what is called "cloud storage" or "online storage". As illustrated in FIG. 13, the data processing unit 320 may perform the above-mentioned identifying, and the identified product attribute information (P1) may be output from the data transmission unit 340. The output product attribute information (P1) may be sent to, for example, a terminal computer and displayed on its display device.

The units constituting the management device 300 (in the example of FIG. 13, the data receiving unit 310, the data processing unit 320, the storage device 330, the attribute information storage unit 331, the data transmission unit, and the like) may be constructed by combining electronic circuits, electric circuits, and independent processing devices, but it is preferable to configure each of these units using a computer and a program executed by the computer. The technology for storing the identification code and product attribute information in association with each other and outputting the product attribute information corresponding to the input identification code (such as the operation of the above-mentioned data processing unit) can refer to conventionally known relational database technology, search technology, and traceability technology. The management device 300 may be the above-mentioned Web server, a server located on a local area network (LAN), a computer accessible from one or more terminal computers, or may be a single terminal computer itself.

In the present invention, when a plurality of items are stored in a manner that they are associated with one another, it means that these items can be used as queries to search for each other's items. For example, in a case where the "identification code" and the "product attribute information" are stored in association with each other, when you search using the "identification code" as a query, the "product attribute information" will be output, and conversely, when you search using the "product attribute information" as a query, the "identification code" will be output.

The product attribute information (P1) of the package is the highest level information that indicates the configuration of the package, and may contain only information about typical items, such as the identification code of the packaging material and the identification code of the production device included in the package, or it may include information necessary for step management and traceability, such as detailed identification codes of the components that make up the production device and product attribute information. The product attribute information (P1) may be input in advance by a data input device.

### (Identification Code and Product Attribute Information of Production Device of Package)

In a preferred embodiment, the management system manages product attribute information of the production device of the package. An identification code (D10) of the production device may be displayed at any portion on the production device to be readable by the reading device 400. Although the identification code of the production device is not shown in FIG. 13, it is preferable to display the identification code at an appropriate position such that it can be distinguished from the identification codes of the components. The identification code (D10) may also be provided on the surface of the packaging material such that the contents can be identified before opening. In this embodiment, the product attribute information (P1) of each of all the packages stored in the attribute information storage unit can include the identification code (D10) of the production device of the corresponding package and the product attribute information (P10) thereof in a mutually associated state. A relationship regarding the product attribute information (P1), the identification code (D10), and the product attribute information (P10), as well as a relationship between the lower level identification code and the product attribute information described below, can be made by referring to conventionally known relational database relationship techniques, such as relationships using tables (including relationships between tables) and correspondences in a matrix format such as (D10, P10). As in the case of the identification code (D1) described above, the management device 300 may be configured to refer to the identification code (D10) received by the data receiving unit 310, and the identification code (D10) and the product attribute information (P10) stored in the attribute information storage unit 331, and output product attribute information (P10) associated with the received identification code (D10). Such a configuration may be realized by the data processing unit 320, the attribute information storage unit 331, and the data transmission unit 340, as described above.

The identification code (D1) of the package and the identification code (D10) of the production device can be assigned by the manufacturer of the package. The manufacturer of the package preferably keeps a record of each product attribute information and detailed management information associated with the identification code (D1) of the package and the identification code (D10) of the production device. Also, it is preferable that the manufacturer of the package record the identification code (D1) and product attribute information (P1) of the package, and the identification code (D10) and product attribute information (P10) of the production device in the attribute information storage unit 331 of the management device 300 via a terminal computer or the like.

In another embodiment of the management system, the identification code (D1) of each package and the identification code (D10) of the production device (closed system portion) of the package may be the same. That is, since the closed system portion is the important item for the cell producer, who is the user of the package, the identification code (D10) of the closed system portion may be used as the identification code of the package when ordering or delivering the package. In this case, the product attribute information (P1) of each of the above-mentioned packages may also be the same as the product attribute information (P10) of each closed system portion, and the identification code and product attribute information of each packaging material may be associated with the identification code or product attribute information of the closed system portion as information about accessory components subordinate to each closed system portion.

### (Attribute Information of Substance Stored in Each Sealed Container)

In a preferred embodiment, the management system manages attribute information of the substances stored in the sealed containers in the production devices of packages. In each production device, an identification code (D20) of each substance stored therein can be displayed on the corresponding sealed container that stores the substance such that it is readable by the reading device 400. Although the identification code of each substance is not shown in FIG. 13, it is preferable to display the identification code at an appropriate position on each sealed container. In this embodiment, the product attribute information (P10) of each production device of the packages includes an identification code (D20) of each substance stored in each sealed container and product attribute information (P20) thereof in a mutually associated state. The management device 300 can be configured to refer to the identification code (D20) received by the data receiving unit 310, and the identification code (D20) and the product attribute information (P20) stored in the attribute information storage unit 331, and output product attribute information (P20) associated with the received identification code (D20). Such a configuration may be realized by the data processing unit 320, the attribute information storage unit 331, and the data transmission unit 340, as described above.

The product attribute information (P20) of a substance preferably includes information necessary for quality management and traceability in the process, such as the name of the substance, the date of production of the substance, the identification code assigned by the manufacturer of the substance at the time of delivery, the date of storage in a sealed container, the identification code of the sealed container, and the expiration date of the substance.

The identification code (D20) of the substance may be assigned by the manufacturer who stores the substance in a sealed container. It is preferable that the manufacturer keeps a record of product attribute information and other information associated with the identification code of the substance. Also, it is preferable that the manufacturer records the identification code (D20) and the associated product attribute information (P20) in the attribute information storage unit 331 of the management device 300 via a terminal computer or the like. It is also preferable that the manufacturer of the substance keeps a record of detailed product attribute information in association with the identification code assigned to the substance at the time of delivery.

### (Product Attribute Information of Components that Constitute Cell Production Device)

In a preferred embodiment, the management system manages product attribute information of components that constitute the production device of the package. Identification codes (D30) may be displayed on the components to be readable by the reading device 400. Although the identification code of each component is not shown in FIG. 13, it is preferable to display the identification code at an appropriate position on the component. In this embodiment, the product attribute information (P10) of each production device in the packages includes identification codes (D30) of the components constituting the production device and the corresponding pieces of product attribute information (P30) in a mutually associated state. The management device 300 is configured to refer to the identification code (D30) received by the data receiving unit 310, and the identification code (D30) and the product attribute information (P30) stored in the attribute information storage unit 331, and output product attribute information (P30) associated with the received identification code (D30). Such a configuration may be realized by the data processing unit 320, the attribute information storage unit 331, and the data transmission unit 340, as described above.

Components to be managed include all components that may directly or indirectly affect cell production, such as packaging materials, sealed containers, connectors, piping members (such as soft tubes), and valves.

The product attribute information (P30) of a component preferably includes information necessary for quality management and traceability in the process, such as the name of the component, the date of production of the component, the identification code assigned by the manufacturer of the component at the time of delivery, and the expiration date of the component (the period during which the sterilization state is maintained).

The identification code (D20) of the component may be one assigned by the manufacturer of the component. It is preferable that the manufacturer of the component keeps a record of product attribute information and other information associated with the identification code. When the component is an assembly having a sealed container, connectors for its inlet and outlet ports, tubes connected to the connectors, and connectors attached to leading ends of the tubes, the manufacturer who assembles such an assembly may assign an identification code to the assembly. Also, it is preferable that the manufacturer or the like who assembles the assembly records the identification code (D20) and the associated product attribute information (P20) in the attribute information storage unit 331 of the management device 300 via a terminal computer or the like. Regardless of the unit into which all the components constituting each package are assembled, preferably, the identification code of each component and its product attribute information are associated with the identification code of the package and are therefore traceable.

### (Attribute Information of Somatic Cells)

In a preferred embodiment, the management system manages attribute information of the somatic cells processed in the production device of the package. The attribute information of the somatic cells to be processed may be input as one of the specification data by the cell producer or the like. In a preferred embodiment, each container storing the solution containing the somatic cells displays a code (D2) of the somatic cells in the container or of the donor thereof such that the code is readable by the reading device. The code of the somatic cells in each container or the donor is a unique code assigned to the somatic cells or the donor, and is synonymous with the above-mentioned identification code (a combination of numbers, letters, symbols, figures, or the like assigned to be unique to each object to be identified). On each container storing a solution containing somatic cells, such as the blood collection tube 30 illustrated in FIG. 13, a code (D2) of the somatic cells in the container or the donor is displayed such that it is readable by the reading device. The code (D2) is assigned, for example, by a medical institution or the like that collected the solution containing the somatic cells. It is preferable that the medical institution or the like keeps a record in which the code (D2) is associated with the attribute information of the donor. When a user (cell producer) is provided with a solution containing somatic cells, the user preferably records the code (D2) and the associated attribute information (P2) in the attribute information storage unit 331 of the management device 300 via a terminal computer 410 or the like. In this case, it is preferable to associate the code (D2) and the attribute information (P2) with the identification code (D1) of the package used in processing the cells or the identification code (D10) of the production device. As a result, the attribute information storage unit 331 stores the codes (D2) and the pieces of attribute information (P2) thereof, and the codes (D2) and the pieces of attribute information (P2) can be associated with each other. Furthermore, the codes (D2) and the pieces of attribute information (P2) thereof can be associated with the identification code (D1) of the package used in the processing or the identification code (D10) of the production device.

The management device 300 may be configured to refer to the code (D2) received by the data receiving unit 310, and the identification code and the attribute information stored in the attribute information storage unit 331, and to output attribute information (P2) associated with the received code (D2), and output an identification code (D1) of the package for use in processing cells or an identification code (D10) of the cell production device in the package. This makes it possible to know, from the code (D2) of a solution containing somatic cells obtained from a patient, which package is to be used for processing the cells, and, from the identification code of a certain package, which patient's somatic-cell containing solution is to be processed using the production device in that package.

Attribute information (P2) of somatic cells (or the donors thereof) includes the collection date, collection hospital, donor code, age, gender, type of solution containing the somatic cells (such as peripheral whole blood and urine), and administration date. However, from the viewpoint of protecting personal information and research ethics, the information that cell producers can obtain is limited to information that does not identify the individual donor of the somatic cells, such as the donor code and gender.

### (Attribute Information of Patient To Whom Produced Differentiated Cells are To Be Administered)

Preferably, the patient to whom the cells produced by the production device of the package are administered is the same as the patient who provided the somatic cells, and the management system enables autotransplantation of the cells. Also, preferably, the patient to whom the cells produced by the production device of the package according to the present invention are administered is the same as the donor of the somatic cells, and this management system reduces management errors such as mix-ups in autotransplantation of the cells. In another preferred embodiment, the management system manages attribute information of a patient to whom differentiated cells produced by the production device of the package are to be administered. It is preferable that a person who knows the attribute information of the patient to whom the differentiated cells will be administered, such as a medical institution requesting the production of differentiated cells, records the code (P3) of the patient to whom the differentiated cells will be administered in the attribute information storage unit 331 of the management device 300 via a terminal computer or the like, in association with the identification code (D1) of the package or the identification code (D10) of the production device. As a result, the attribute information storage unit 331 may store the code (P3) of the patient to whom the cells produced by the production device are to be applied. The code (P3) of the patient to whom the cells are to be applied may be associated with the identification code (D1) of the package for use in processing or the identification code (D10) of the production device.

The management device 300 is configured to refer to the identification code (D1) or the identification code (D10) received by the data receiving unit 310, and the identification code and the attribute information stored in the attribute information storage unit 331, and output the code (P3) of the patient to whom the produced cells are to be applied. This makes it possible to clearly identify the relationship between the package or production device for use in processing and an intended patient in autotransplantation or allogeneic transplantation, and the produced cells can be administered to the intended patient without being confused.

As described above, known cell processing devices (such as CliniMACS Prodigy available from MiltenyiBiotec, Gibco CTS Rotea Counterflow Centrifugation System available from Thermo Fisher Scientific, Cocoon system available from Lonza, and Xuri available from Cytiva) are constituted by a closed system processing circuit portion, a mechanism that operates the processing circuit portion, and a control unit. The closed system processing circuit portion is detachable from the mechanism that operates the processing circuit portion and control unit, and serves as a replaceable unit for each cell processing run. In a preferred embodiment of the present invention, the cell production device of the package is provided as a detachable closed system processing circuit portion in a known cell processing device described above.

In this case, of the known cell processing devices, the device body (such as mechanism portions and housing portions) excluding the closed system processing circuit portion is given an identification code (D11) of the cell processing device, and the identification code (D11) is displayed such that it is readable by a reading device. As a result, the known cell processing devices are also to be managed by the management device 300. The attribute information storage unit 331 of the management device 300 stores the identification code (D11) of the cell processing device and the product attribute information (P11) thereof by being input in advance. Examples of product attribute information (P11) include the name of the cell processing device, the name of the manufacturer, the product number, and the management number. The identification code (D11) of the cell processing device and the product attribute information (P11) thereof are stored in the attribute information storage unit 331 in association with each other and in association with the identification code (D1) of the package for use in processing or the identification code (D10) of the cell production device of the package. The management device 300 is configured to refer to the identification code (D1) or identification code (D10) received by the data receiving unit and the identification code (D11) and the product attribute information (P11) stored in the attribute information storage unit, and to output an identification code (D11) and product attribute information (P11) thereof. Such a configuration may be realized by the data processing unit 320, the attribute information storage unit 331, and the data transmission unit 340, as described above. This makes it possible to track which cell processing device was used in managing the cell production step.

### (Checking Specifications of Package through Management System)

In a preferred embodiment, the management system manages whether the package has been produced according to specifications. In this embodiment, the production device of the package according to the present invention may be formed as a detachable closed system processing circuit portion in a cell processing device such as that shown in FIG. 16. As described above, examples of such cell processing devices include CliniMACS Prodigy available from MiltenyiBiotec, Gibco CTS Rotea Counterflow Centrifugation System available from Thermo Fisher Scientific, Cocoon system available from Ronza, and Xuri available from Cytiva. However, they may be cell processing devices produced for the present invention. Such a cell processing device may include a detachable closed system processing circuit portion, a mechanism that operates the processing circuit portion, and a control unit. In this embodiment, the production device of the package may be configured as the detachable closed system processing circuit portion. As illustrated in FIG. 13, the control unit of the cell processing device 500 (the internal configuration of the cell processing device is not shown) as described above may be connected to the management device 300 via the Internet to be able to perform data communications.

In the preferred embodiment described above, the management device 300 may further include a specification data storage unit 332. The management device 300 can have the specification data storage unit 332 in the storage device 330. The specification data storage unit 332 can store the specification data (S1a) of all the packages to be produced. As will be described later, the specification data (S1a) is created by the user, who is the cell producer, according to the cells to be produced, and may include the following items. However, from the viewpoint of protecting personal information and research ethics, the attribute information of the donor of the solution containing somatic cells used in cell production is limited to information that does not identify the individual donor of the somatic cells, such as the code and gender of the donor, which the cell producer can obtain.

Examples thereof include: the attribute information of a solution containing somatic cells (such as blood or urine) for use in cell production or a donor thereof;
product attribute information of the package or production device (information about the substance of each sealed container connected to the processing container);
the date when the production device is used (the date when cells are produced);
identification information (such as device number or management number) of the cell processing device on which the production device is installed as the processing circuit portion; and
production type (serial number).

In the preferred embodiment described above, the management system can have a data input device for inputting the specification data (S1a). As illustrated in FIG. 13, the terminal computer 410 can function as the data input device. In the above-described embodiment, all the packages to be produced can be assembled based on the respective specification data (S1a).

The specification data (S1a) can be created by the user of the package, who is the cell producer, depending on the cells to be produced. The created specification data (S1a) can be stored in advance in the specification data storage unit 332 of the management device 300 via a data input device.

The user of the package can send the specification data (S1a) to the manufacturer of the package via the Internet or the like. The manufacturer of the package can send part or all of the specification data (S1a) to the manufacturers of the individual components via the Internet or the like. Alternatively, the user of the package may simultaneously send part or all of the specification data (S1a) to the manufacturer of the package and the manufacturer of each component.

The manufacturer of each component can produce the component based on the specification data (S1a). It is preferable that the manufacturer of each component marks the component with an identification code and keeps a record of the identification code and pieces of product attribute information to ensure traceability. Also, it is preferable that the manufacturer of each component records the identification code and the product attribute information in the attribute information storage unit 331 of the management device 300 via a terminal computer or the like. The manufacturer of each component delivers the produced component to the manufacturer of the package.

The manufacturer of the package can produce the package based on the specification data (S1a) using the delivered components. It is preferable that the manufacturer of the package marks the package and the production device with identification codes and keeps records of the identification codes and pieces of product attribute information to ensure traceability. Also, it is preferable that the manufacturer of the package records the identification codes and pieces of product attribute information of the package and the production device in the attribute information storage unit 331 of the management device 300 via a terminal computer or the like. The manufacturer of the package can deliver the produced package to the user (cell producer).

In the preferred embodiment described above, the management device 300 can keep the current date through data communication with a time server on the Internet or the like.

On the day of cell production, the user can input actual specification data (S1b) including items corresponding to the previously input specification data (S1a) into the management device 300 via the data input device. The actual specification data (S1b) includes at least attribute information of the solution containing somatic cells for use in cell production or the donor thereof, product attribute information of the package or production device for use in cell production, the date of the day, and identification information of the cell processing device used.

The management device 300 can receive the input specification data (S1b) at the data receiving unit.

The management device 300 may be configured to compare the specification data (S1b) received by the data receiving unit with the specification data (S1a) stored in advance in the specification data storage unit, and may be configured to output a signal indicating that these sets of data match each other when they match. The terminal computer may be configured to upon receiving this signal output, display a symbol or character indicating a "match" on a display device, or may be configured to make a sound indicating a "match", or may be configured to notify the user of the match. This prevents the user from using a production device in a package that differs from the specification data (S1a) when producing cells, and can also inhibit the user from making a mistake in the production date. Furthermore, it is possible to inhibit the user from using a solution containing somatic cells that differ from the specification data (S1a).

In a more preferred embodiment, as illustrated in FIG. 13, a control unit (not shown) of the cell processing device 500 for use and the management device 300 can be connected to be able to perform data communications. The control unit of the cell processing device 500 may be configured to receive the above-mentioned "signal indicating a match" output from the management device 300, and may be configured to enable the cell processing device 500 to start only when the "signal indicating a match" is received. "Enabling the cell processing device 500 to start" means that the cell processing device 500 is not started immediately upon receiving the "signal indicating a match", but rather the user is allowed to start the cell processing device 500, for example, by enabling the input of a start signal to start the cell processing device 500. This inhibits the user from using a production device of a package that differs from the specification data (S1a) when producing cells, and can also inhibit the user from making a mistake in the production date. Furthermore, it is possible to inhibit the user from using a solution containing somatic cells that differ from the specification data (S1a).

As described above, in a preferred embodiment of the production device of the package according to the present invention, a sensing probe is provided at a leading end of the processing container or the discharge side of the discharge port 102, and the oxygen concentration and pH of the contents are measured by the sensing probe. Data on the oxygen concentration and pH of the contents measured by such sensors as sensing probes may be associated with, for example, product attribute information (P10) of the production device, and input and stored in the attribute information storage unit 331 of the management device 300 or other data storage unit. As a result, the management system according to the present invention also functions as a system for recording and managing producing conditions during cell production.

### (Preferred Embodiment for Providing Package to Cell Producer)

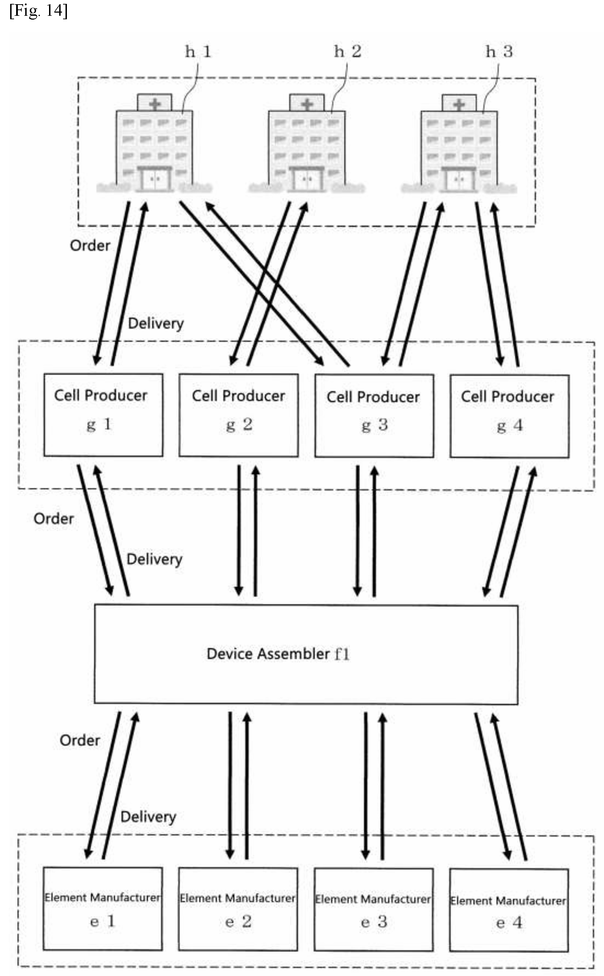

FIG. 14 is a block diagram illustrating the flow of orders and delivery in the step of producing a package. In FIG. 14, symbols h1 to h3 indicate medical institutions that perform cell therapy such as autotransplantation. Cell producers g1 to g4 are manufacturers that process cells using the cell processing device, and element manufacturers e1 to e4 are specialists that produce each element (including detailed components, units assembled from components, and the like) that constitute the closed system portion, divided by field. Although not shown in FIG. 14, the element manufacturers may order and obtain the components included in the element to be produced, from another element manufacturer.

The block diagram illustrated in FIG. 14 also shows a method for providing a package (or a method for producing the package). As illustrated in FIG. 14, in the method for supplying the package (or the method for producing the package), a device assembler f1 is placed between cell producers g1 to g4 and element manufacturers e1 to e4, the supplying method including:
a step of the device assembler placing an order for an element and/or component with a predetermined element manufacturer based on specification data regarding a closed system portion issued by the cell producer; and
a step of the device assembler assembling the closed system portion using the element and/or component delivered by the element manufacturer in response to the order and based on the specification data, packaging the closed system portion into the package, and supplying (delivering) the package to the cell producer.

The cell producer sets the supplied closed system portion in the device body of a predetermined cell processing device to produce cells. As will be described later, the specification data regarding the closed system portion is determined by the cell producer in response to an order for cells received from a medical institution.

The numbers of medical institutions, cell producers, device assemblers, and element manufacturers are not limited to those shown in FIG. 14, and may be one or more. Since the closed system portion includes multiple elements and the materials and components that constitute these elements (such as liquid culture media, reagents, containers (bags), connecting conduits, and joints) span multiple fields, there are usually multiple element manufacturers. There may also be multiple medical institutions performing autotransplantation and multiple cell producers that produce cells for autotransplantation. The number of device assemblers and element manufacturers may be increased or reduced as appropriate depending on the demand for the closed system portion.

The cell producer and the device assembler may each be an individual, a company, or an organization, and are preferably different business entities. The different business entities may be different business groups or divisions with different profitability within the same company or corporation, or may be independent companies or corporations. In particular, when the cell producer and the device assembler are independent companies or corporations, from the viewpoint of cell producers, there is no need for facilities or personnel to prepare reagents and other items in advance, allowing them to concentrate on cell production, reducing risk and making it easier to enter the cell producing business. From the viewpoint of market formation in regenerative medicine, each company can divide up roles in their area of expertise, allowing more companies to enter the market. From the viewpoint of element manufacturers, the market is still in the process of being formed, and thus production lot sizes are likely to be small and the unit price of package will be high. However, by establishing a system where device assemblers can produce and sell packages that can be delivered to multiple cell producers, it will be possible to secure a certain number of lots. Furthermore, from the viewpoint of patients and medical institutions, this system has the advantage of reducing total medical costs.

The flow from an order for cells by a medical institution to delivery of the cells to the medical institution is carried out in the following order, for example, along the arrows in FIG. 14.
(1) A medical institution (e.g., h1) that performs autotransplantation orders the cells necessary for patient treatment from a cell producer (e.g., g1). At this time, the medical institution h1 notifies the cell producer g1 of the identification code of the patient. Furthermore, the medical institution h1 collects somatic cells necessary for cell production from the patient using a blood collection device or the like, which will be described later, and sends the blood collection device or the like storing the somatic cells to the cell producer g1.
(2) Upon receiving an order from the medical institution h1, the cell producer g1 selects a cell processing device suitable for producing the ordered cells, determines the configuration of each portion of the closed system portion used in the cell processing device, and creates specification data that defines the configuration.
(3) The cell producer g1 orders the device assembler f1 to produce the closed system portion, and issues (sends) the created specification data of the closed system portion to the device assembler f1. This issuance may be performed by uploading the specification data to a shared folder in a management device (a server on the Internet) and notifying the user thereof, or by directly transmitting the specification data.
(4) The device assembler f1 places orders for the elements to be produced in a shared manner with each of the necessary element manufacturers e1 to e4, and also sends the specification data. This sending of the specification data may be performed by uploading the specification data to a shared folder in the management device and notifying the user of this, or by directly transmitting the specification data. The sending of the specification data may also serve as an order. The specification data may be sent directly from the cell producer g1 to the element manufacturer. Each element manufacturer may receive the overall specification data issued by the cell producer g1, or may receive specification data for each component to be produced.
(5) Each of the element manufacturers e1 to e4 produce the elements that they need to produce based on the specification data. The produced elements display identification codes associated with the product attribute information. Each identification code may be a two-dimensional symbol such as a QR code (registered trademark). The elements are delivered in sterile packages to the device assembler f1.
(6) The device assembler f1 receives the necessary elements from each of the element manufacturers e1 to e4 and assembles the closed system portion according to the specification data. A blood collection device storing somatic cells and the like are also incorporated in the closed system portion. The assembled closed system portion displays the above-mentioned identification code (D10) for the closed system portion, which is associated with the product attribute information or identification codes of all the elements used. This identification code (D10) contains the identification code of the patient. The identification code is preferably a two-dimensional symbol such as a QR code (registered trademark) (the same applies to the other identification codes described below).
(7) The device assembler f1 aseptically packages the assembled closed system portion in a packaging material to produce a package according to the present invention, and delivers the package to the cell producer g1. The package displays an identification code for the closed system portion or an identification code of the package associated therewith.
(8) The cell producer g1 attaches the closed system portion of the package to the device body of the cell processing device and produces the target cells.
(9) The cell producer g1 marks a sealed container storing the produced cells with an identification code containing information about the cells, and sends the sealed container to the medical institution h1 in a sterile manner. The information about the cells includes information about the identity of the cells, the production history of the cells (such as the production date, factors involved in the production, and information about the cell processing device), and information about the somatic cells, which serve as the raw material, (information including the identification code of the patient). At this time, it is essential that [(information about the target cells, patient identification code (= somatic cell information)) received when the order was first placed from the medical institution h1] and [(information about the produced cells, information about the somatic cells used as raw materials (= patient identification code)) contained in the identification code displayed on the sealed container storing the cells to be delivered to medical institution h1] at least match each other, and that the above information is correctly attached to the bag. The consistency of this information makes it possible to perform cell therapy without mistakes such as mix-ups.

The blood collection device and the like in (1) of the logistics step may be, for example, the blood collection device illustrated in FIG. 6, but may also be a sealed container that can store collected somatic cells and is connectable to the closed system portion. The blood collection device and the like may be contained in a package, in which case the blood collection device and the like may be sent from the cell producer g1 to the medical institution h1. The blood collection device and the like may be held in advance by the medical institution. In either case, the cell producer g1 who receives the order will inform the medical institution h1 of the date and time of cell production. The medical institution h1 collects somatic cells from the patient using the blood collection device and the like such that the somatic cells can be provided on the date and time of cell production, and marks the blood collection device and the like with the identification code of the patient and necessary information (such as that the cells are collected from peripheral blood or that they have been collected from a specific area), and sends the cells to the cell producer in a sterile manner.

It is preferable that the specification data include at least information about the donor of the somatic cells (especially the identification code of the patient), information about the cell processing device used, information about the configuration of the closed system portion, and the date of use of the closed system portion (date and time when cell production is started). The specification data includes specifications for each element such as the culture medium container and the first supply container, specifications for the connecting conduits, and preferably also includes specifications regarding the arrangement of where the culture medium container and the first supply container are connected to the connecting conduits.

In (6) of the logistics step, the identification code is displayed on the assembled closed system portion, and through this identification code, information about the donor of the somatic cells contained in the specification data and information about the actual configuration of the produced cell production device can be obtained from the separately provided management device mentioned above or the process management device described below. It is preferable that information about the actual configuration of the produced closed system portion is associated with each item of information about the configuration of the closed system portion included in the specification data and sequentially recorded in the storage device of the management device.

### 4. Quality Management System for Closed System Portion in Package

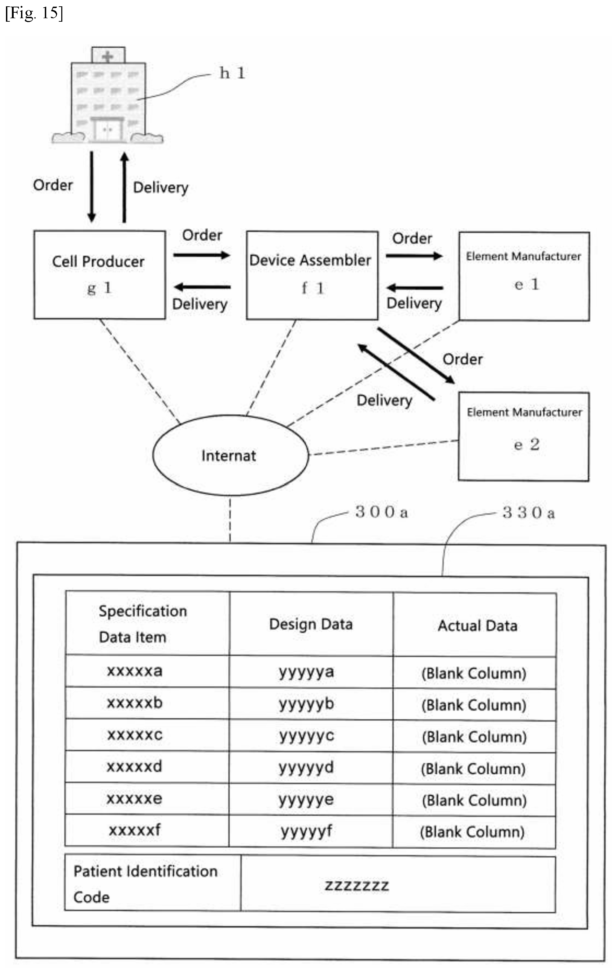

Next, a quality management system according to the present invention (hereinafter also referred to as the quality management system) will be described with reference to a process chart illustrated in FIG. 14 and a block diagram illustrated in FIG. 15. The quality management system is a system for managing a step of producing a closed system portion (cell production device) included in a package according to the present invention, and for managing the quality of the closed system portion.

As described above using FIG. 14, in the package producing step, a device assembler is placed between the cell producer and the element manufacturer, and the producing step includes an element producing step and a device assembly step. In the element producing step, for each of a plurality of elements that constitute the closed system portion in the package is produced by an element manufacturer in the respective technical field of the element. In the device assembly step, the elements produced in the element producing step are collected and the device assembler assembles the elements into the cell production device. The quality management system records data on orders and products (elements, closed system portion) in each step, clarifies the history of the somatic cells (raw material), materials, and components involved in the cells to be produced, and increases the reliability of the cells.

As illustrated in FIG. 15, the quality management system includes a process management device 300a and a plurality of terminal devices (not shown). In FIG. 15, for simplicity of illustration, the number of medical institutions, the number of cell producers, and the number of device assemblers are each set to 1, and the number of element manufacturers is set to 2 (e1, e2).

The plurality of terminal devices are connected to the process management device to enable data communication. Although the terminal devices are not shown in FIG. 15, each of the plurality of terminal devices is arranged to be used by the cell producer g1, the device assembler, and the element manufacturers. This allows the cell producer, device assembler, and element manufacturer to communicate with the process management device 300a as shown in FIG. 15.

As described above, the closed system portion is assembled based on the specification data that indicates the configuration. The specification data was issued by the cell producer g1. After receiving an order from the medical institution h1 to produce target cells for patient treatment, the cell producer selects a cell processing device according to the production of the target cells and designs the specification of the closed system portion. Therefore, the specification data includes information about the selected cell processing device, and is designed such that the closed system portion has a configuration for producing the target cells and is adapted to the device body of the cell processing device. The specification data is associated with the identification code of the patient. The specification data is preferably associated with information about the somatic cells (such as information indicating that the cells are from the peripheral blood of the patient).

The specification data is stored in the storage device 330a of the process management device 300a. In the example shown in FIG. 15, the specification data is stored as a table in a predetermined storage area of the storage device 330a. In the table, the specification data items (xxxxxa to xxxxxf) are associated with the design data (yyyyya to yyyyyf) that defines the configuration of each item in order. Also, the specification data stored in the table is associated with the identification code (zzzzzzz) of the patient. The design data (yyyyya to yyyyyf) may be the product attribute information described above.

The process management device 300a receives specification data and the patient identification code associated therewith from the cell producer g1 or the device assembler f1 via their respective terminal devices, and stores them in the storage area 330a.

Furthermore, in the process management device 300a, a storage area for storing actual data is secured. This actual data corresponds to each item of the specification data in each configuration of the closed system portion that is actually produced. The storage area may be automatically secured or may be created by the cell producer g1 or the device assembler f1. In the example shown in FIG. 15, the storage area for actual data is provided in the table above as blank columns (areas where no data is written) corresponding to the specification data items (xxxxxa to xxxxxf), but the manner in which each data is associated and stored is not limited.

The element manufacturers (e1, e2) that receive production orders from the device assembler f1 produce elements based on the specification data and transmits the actual data of each element to the process management device 300a. The process management device 300a receives the actual data of each element and stores it in a storage area for actual specification data (the blank "actual data" column shown in FIG. 15).

The device assembler f1 assembles the closed system portion using the elements delivered from the element manufacturers (e1, e2). The device assembler f transmits data regarding the overall arrangement of the assembled closed system portion to the process management device 300a. The process management device 300a receives the data regarding the overall arrangement and stores it in a storage area for actual specification data (the blank "actual data" column shown in FIG. 15). This ensures that all actual data corresponding to the specification data is recorded.

In the above state, the process management device 300a determines whether the original specification data and the actual specification data match, and outputs the determination result. This allows the device assembler f1 and the cell producer g1 to confirm that the assembled closed system portion has the configuration ordered by the cell producer g1.

The step management system may utilize the configuration of the management system described above, and the process management device 300a shown in FIG. 15 can utilize the management device 300 shown in FIG. 13. Each of the terminal devices can utilize the terminal computer 410 shown in FIG. 13. The terminal computer is preferably a tablet computer or the like, and preferably has the function of reading two-dimensional symbols such as a QR code (registered trademark).

The configuration of data management shown in FIG. 15 is one example, and data indicating the configuration of each produced element may be recorded in a two-dimensional symbol and displayed on each element.

It is preferable that an identification code indicating the respective pieces of identification information are displayed on the surface of the packaging material of the package, on a representative portion of the closed system portion, and on each element. In particular, if the identification code is a two-dimensional symbol such as a QR code (registered trademark), it can include the URL of a process management device provided as a server computer on the Internet. This makes it possible to easily access the table stored in the storage device of the process management device using the two-dimensional symbol and the reading function of the terminal device.

### Industrial Applicability

The package according to the present invention and the method for producing cells using the package eliminate a conventional step of aseptically preparing many types of substances and containers, and significantly reduce the operation of connecting many types of substance supply bags to predetermined ports when the cell processing device is in operation, thereby reducing the occurrence of incorrect substance supply bags, incorrect installation positions, and/or incorrect connections of soft tubes. This has reduced cell production errors and made it possible to stably obtain the target cells.

The management system according to the present invention allows the step of producing the package according to the present invention to be preferably managed, and inhibit packages that do not meet specifications from being used.

By appointing the device assembler between the cell producer and the element manufacturer and using a quality management system managed by the computer, the cell producer can obtain the closed system portion on the day of cell production or an appropriate date before that, and is free from the hassle and errors involved in assembling the closed system portion.

The present application claims the benefit of priority based on Japanese Patent Application No. 2023-184314 (filing date: October 26, 2023) and Japanese Patent Application No. 2024-117090 (filing date: July 22, 2024) filed in Japan, which are incorporated herein by reference in their entirety.

### Reference Numerals

- 1: Package
- 10: Cell production device
- 20: Packaging material
- 100: Processing container
- 101 to 105: Port for processing container
- 210: Culture medium container
- 220: First supply container
- 230: Second supply container

## Claims

1. A package of a cell production device comprising:
a cell production device; and
a packaging material,
wherein the cell production device has, as a configuration for producing induced pluripotent stem cells,
a processing container,
at least one culture medium container for storing a liquid culture medium, and
a first supply container for storing a fluid containing a reprogramming factor,
the culture medium container and the first supply container are, such that their contents are fed into the processing container while maintaining a closed system,
(i) connected to the processing container via a connecting conduit which is switchable between a communication state and a non-communication state, or
(ii) connectable to the processing container via the connecting conduit and via a unique connector configured to prevent incorrect interconnection,
the processing container has at least an injection port that is openable and closable for receiving a solution containing somatic cells from an outside, and a discharge port that is openable and closable for discharging the contents,
the processing container, the culture medium container, and the first supply container are sealed containers, and
the cell production device is packaged in the packaging material in a sterilized state.

2. The package of a cell production device according to claim 1,
wherein the processing container is
configured to separate somatic cells for use in production of induced pluripotent stem cells from the solution containing somatic cells injected through the injection port, and
configured to discharge components other than the somatic cells to the outside of the processing container, and leave the somatic cells to be processed inside the processing container, and
the somatic cells remaining inside the processing container are processed inside the processing container.

3. The package of a cell production device according to claim 2,
wherein the processing container is configured to separate somatic cells for use in production of induced pluripotent stem cells through continuous centrifugation.

4. The package of a cell production device according to any one of claims 1 to 3,
wherein the cell production device has one or more second supply containers for storing a fluid containing a differentiation-inducing substance for the induced pluripotent stem cells as a configuration for producing differentiated cells from the induced pluripotent stem cells produced in the processing container,
the second supply containers are sealed containers,
the second supply containers are, such that their contents are fed into the processing container while maintaining a closed system,
(iii) connected to the processing container via a connecting conduit which is switchable between a communication state and a non-communication state, or
(iv) connectable to the processing container via the connecting conduit which is switchable between a communication state and a non-communication state and via a unique connector configured to prevent incorrect interconnection.

5. The package of a cell production device according to any one of claims 1 to 4,
wherein one or both of the culture medium container and the first supply container are each separated from the cell production device and packaged in separate packaging materials, and the package is separated into two or more packages that are independent of each other.

6. The package of a cell production device according to any one of claims 1 to 5,
wherein the cell production device packaged in the packaging material in the package is a closed system processing circuit portion included in the cell processing device for processing the somatic cells,
the cell processing device is configured to include the closed system processing circuit portion and a device body,
the device body includes an operating mechanism that operates the closed system processing circuit portion and a control unit that controls the operating mechanism, and
the closed system processing circuit portion is detachable from the device body while maintaining the closed system, and is assembled as a closed system in a state in which the processing circuit portion is taken out from the device body, and the closed system processing circuit portion attached to the device body is operated by the operating mechanism under control of the control unit, thereby processing the somatic cells inside the closed system processing circuit portion.

7. The package of a cell production device according to claim 6,
wherein the cell production device is assembled as a closed system by a device assembler separate from a cell producer who produces cells using the cell processing device, and the cell production device is assembled based on specification data issued by the cell producer, packaged as the package, and provided to the cell producer.

8. The package of a cell production device according to claim 7,
wherein the cell producer and the device assembler are different entities.

9. The package of a cell production device according to claim 7 or 8,
wherein the specification data includes
at least information about a donor of the somatic cells,
information about the device body to which the cell production device is to be attached as a closed system processing circuit portion,
information about a configuration of the cell production device, and
the date of use of the cell production device,
the cell production device displays an identification code that is readable by a reading device, and information about the donor of the somatic cells included in the specification data and information about an actual configuration of the cell production device produced are obtainable from a separately provided management device through the identification code, and
the information about the actual configuration of the cell production device is stored in a storage device of the management device in association with information about the configuration of the cell production device included in the specification data.

10. A cell production method using the package of a cell production device according to any one of claims 1 to 9, the cell production method comprising:
a step of preparing the cell production device taken out from the package; and
a step of producing induced pluripotent stem cells in the processing container of the cell production device,
wherein the step of producing the induced pluripotent stem cells includes
a step (s10) of injecting the solution containing the somatic cells into the processing container through the injection port, feeding the liquid culture medium from the culture medium container into the processing container, and feeding the fluid containing the reprogramming factor from the first supply container into the processing container, thereby bringing the reprogramming factor into contact with the somatic cells in the liquid culture medium in the processing container, and
a step (s20) of culturing the somatic cells in the liquid culture medium in the processing container to establish induced pluripotent stem cells (s20).

11. The cell production method according to claim 10,
wherein the package of a cell production device is the package according to claim 2 or 3,
in the step (s10),
the solution containing the somatic cells is injected into the processing container through the injection port,
then the somatic cells for use in production of induced pluripotent stem cells are separated in the processing container, and components other than the somatic cells are discharged to an outside of the processing container, the somatic cells to be processed are left inside the processing container, and
then the liquid culture medium is fed from the culture medium container, and the fluid containing the reprogramming factor is fed from the first supply container, thereby bringing the reprogramming factor into contact with the somatic cells in the liquid culture medium within the processing container.

12. The cell production method according to claim 11,
the package of a cell production device being the package of a cell production device according to claim 4,
the cell production method further comprising, after the step (s20), a step (s30) of feeding the fluid containing the differentiation-inducing substance for induced pluripotent stem cells from the second supply containers into the processing container, thereby forming differentiated cells in the processing container.

13. A management system for the package of a cell production device according to any one of claims 1 to 9,
the management system comprising:
a management device; and
a reading device,
wherein one or both of the cell production device and the packaging materials surrounding the cell production device in all the packages produced displays an identification code (D1) of each package so as to be readable by the reading device,
the reading device is for reading the identification code,
the management device has a data receiving unit and an attribute information storage unit,
the data receiving unit receives the identification code read by the reading device as input data,
the attribute information storage unit stores the respective identification codes (D1) of all the packages to be produced and respective pieces of product attribute information (P1) in a mutually associated state, and
the management device is configured to refer to the identification code (D1) received by the data receiving unit, and the identification codes (D1) and the pieces of product attribute information (P1) stored in the attribute information storage unit, and output product attribute information (P1) associated with the read identification code (D1).

14. The management system according to claim 13,
wherein an identification code (D10) of the cell production device is displayed on the cell production device to be readable by the reading device,
the product attribute information (P1) of each of all the packages stored in the attribute information storage unit includes the identification code (D10) of the cell production device of the package and product attribute information (P10) thereof in a mutually associated state, and
the management device is configured to refer to the identification code (D10) received by the data receiving unit, and the identification code (D10) and the product attribute information (P10) stored in the attribute information storage unit, and output product attribute information (P10) associated with the received identification code (D10).

15. The management system according to claim 14,
wherein each sealed container for storing a substance in the cell production device displays an identification code (D20) of the substance stored in the sealed container such that the identification code (D20) is readable by the reading device,
the product attribute information (P10) of each cell production device in the packages includes an identification code (D20) of each substance stored in each sealed container and product attribute information (P20) thereof in a mutually associated state, and
the management device is configured to refer to the identification code (D20) received by the data receiving unit, and the identification codes (D20) and the pieces of product attribute information (P20) stored in the attribute information storage unit, and output product attribute information (P20) associated with the received identification code (D20).

16. The management system according to claim 14 or 15,
wherein components constituting the cell production device display identification codes (D30) of the corresponding components such that the identification codes are readable by the reading device,
the product attribute information (P10) of cell production devices in the packages includes identification codes (D30) of the components constituting the cell production device and pieces of product attribute information (P30) thereof in a mutually associated state, and
the management device is configured to refer to the identification code (D30) received by the data receiving unit, and the identification codes (D30) and the pieces of product attribute information (P30) stored in the attribute information storage unit, and output product attribute information (P30) associated with the received identification code (D30).

17. The management system according to any one of claims 14 to 16,
wherein each container storing the solution containing somatic cells to be processed in the cell production device displays a code (D2) of the somatic cells in the container or a donor thereof such that the code is readable by the reading device,
the attribute information storage unit stores the code (D2) and the corresponding attribute information (P2), and the code (D2) and the corresponding attribute information (P2) are associated with each other and with an identification code (D1) of a package for use in processing or an identification code (D10) of the cell production device in the package, and
the management device is configured to refer to the code (D2) received by the data receiving unit, and the codes and the attribute information stored in the attribute information storage unit, output the attribute information (P2) associated with the received code (D2), and output the identification code (D1) of the package for use in processing or the identification code (D10) of the cell production device in the package.

18. The management system according to any one of claims 14 to 17,
wherein the attribute information storage unit stores a code (D3) of a patient to whom cells produced by the cell production device need to be applied and attribute information (P3) thereof, and the code (D3) of the patient and the attribute information (P3) thereof are associated with each other and with an identification code (D1) of a package for use in processing or an identification code (D10) of the cell production device in the package, and
the management device is configured to refer to the identification code (D1) or the identification code (D10) received by the data receiving unit, and the identification code and the attribute information stored in the attribute information storage unit, and output the code (D3) of the patient to whom the produced cells are to be applied and the attribute information (P3) thereof.

19. The management system according to any one of claims 14 to 18,
wherein the cell production device in the package is a detachable closed system processing circuit portion in a cell processing device including the detachable closed system processing circuit portion, a mechanism that operates the processing circuit portion, and a control unit,
an identification code (D11) of the cell processing device is displayed on a portion of the cell processing device excluding the closed system processing circuit portion to be readable by a reading device,
the attribute information storage unit stores the identification code (D11) of the cell processing device and product attribute information (P11) thereof,
the identification code (D11) of the cell processing device and the product attribute information (P11) thereof are stored in the attribute information storage unit in association with each other and in association with the identification code (D1) of the package for use in processing or the identification code (D10) of the cell production device in the package, and
the management device is configured to refer to the identification code (D1) or the identification code (D10) received by the data receiving unit, and identification codes and product attribute information stored in the attribute information storage unit, and to output an identification code (D11) and product attribute information (P11) thereof.

20. The management system according to any one of claims 13 to 19,
wherein the cell production device in the package is a detachable closed system processing circuit portion in a cell processing device including the detachable closed system processing circuit portion, a mechanism that operates the processing circuit portion, and a control unit,
the management device further includes a specification data storage unit, the specification data storage unit stores specification data (S1a) including at least
attribute information of the solution containing somatic cells for use or the donor,
product attribute information of the package for use or the cell production device thereof,
the date of use of the cell production device, and
identification information of the cell processing device in which the cell production device is installed as the processing circuit portion, and,
the management system includes a data input device for inputting the specification data, and
the package is assembled based on the specification data (S1a),
the management device is configured to receive, via the data receiving unit,
specification data (S1b) including at least
attribute information of the solution containing somatic cells for use or the donor,
product attribute information of the package for use or the cell production device thereof,
information about the date when the cells are produced, and
identification information of the cell processing device for use, the attribute information, the product attribute information, the information about the date, and the identification information being entered through the reading device by the cell producer on the date when the cells are produced,
and,
to output a signal indicating a match when the specification data (S1b) received by the data receiving unit matches the specification data (S1a) stored in the specification data storage unit.

21. The management system according to claim 20,
wherein the control unit of the cell processing device is configured to receive a signal indicating the match output from the management device, and is configured to enable start of the cell processing device only when the signal is received.

22. A quality management system for the cell production device in the package according to any one of claims 7 to 9,
wherein a process for producing the package includes
an element producing process for each of a plurality of elements constituting the cell production device in the package, in which each element is produced by an element manufacturer in the respective technical field of the element, and
a device assembly process in which the elements produced in the element producing process are collected and the device assembler assembles the elements into the cell production device,
the quality management system includes
a process management device and
a plurality of terminal devices connected to the process management device to enable data communication,
the terminal devices are arranged to be used by a cell producer who is a user of the cell production device, the device assembler, and the element manufacturer,
the cell production device is assembled based on specification data indicating a configuration of the cell production device,
the specification data is designed by the cell producer, who has received a production order from a medical institution to produce target cells for patient treatment, such that the cell production device has a configuration for producing the target cells and is adapted to the cell processing device selected to produce the target cells, and
the specification data is associated with an identification code of the patient,
the process management device
receives the specification data and the identification code of the patient associated therewith from the cell producer or the device assembler via the terminal device and stores them in a storage area,
allocates a storage area for storing actual specification data corresponding to the specification data in the cell production device that is actually produced,
receives, from the element manufacturer, actual data for each element produced by the element manufacturer who has received a production order from the device assembler based on the specification data, and stores the actual data in the storage area for storing the actual specification data,
receives data regarding an overall arrangement of the cell production device from the device assembler who has assembled the cell production device using each element delivered from the element manufacturer and stores the data in the storage area for storing the data, and
determines whether the original specification data and the actual specification data match, and outputs the determination result.

23. The quality management system according to claim 22,
wherein the terminal devices are each equipped with a function of reading an identification code, and
identification codes indicating respective pieces of identification information are respectively displayed on surfaces of the packaging materials of the packages and on the elements of the cell production device.

24. The quality management system according to claim 23,
wherein the identification codes are each displayed in the form of a two-dimensional symbol.

25. The quality management system according to any one of claims 22 to 24,
wherein the cell producer, the device assembler, and the element manufacturer are different entities.
